# EUROPÄISCHE PATENTSCHRIFT

(11) **EP 3 136 993 B1**
(45) Veröffentlichungstag und Bekanntmachung des Hinweises auf die Patenterteilung: **17.07.2019**
(21) Anmeldenummer: 15720935.4
(22) Anmeldetag: 24.04.2015
(51) Int. Cl.: A61B 17/17, A61B 34/00, A61B 90/00

(54) **VORRICHTUNG ZUM ANBRINGEN EINES POSITIONIERUNGSMITTELS AN EINEM KNOCHEN EINES PATIENTEN, VORRICHTUNG ZUM BEARBEITEN EINES KNOCHENS EINES PATIENTEN UND HÜFTIMPLANTATSYSTEM**
DEVICE FOR ATTACHING A POSITIONING MEANS ON A BONE OF A PATIENT, DEVICE FOR MACHINING A BONE OF A PATIENT, AND HIP IMPLANT SYSTEM
DISPOSITIF PERMETTANT DE PLACER UN MOYEN DE POSITIONNEMENT SUR UN OS D'UN PATIENT, DISPOSITIF POUR RECTIFIER UN OS D'UN PATIENT ET SYSTÈME D'IMPLANT POUR LA HANCHE

(30) Priorität: 02.05.2014 DE 102014208283
(43) Veröffentlichungstag der Anmeldung: 08.03.2017
(73) Patentinhaber: Peter Brehm Holding GmbH & Co. KG, 91085 Weisendorf (DE)
(72) Erfinder: BREHM, Peter, 91085 Weisendorf (DE)
(74) Vertreter: Grünecker Patent- und Rechtsanwälte PartG mbB
(86) Internationale Anmeldenummer: PCT/EP2015/058933
(87) Internationale Veröffentlichungsnummer: WO 2015/165817

(56) Entgegenhaltungen:
- US-A1- 2005 107 799
- US-A1- 2006 161 167

## Beschreibung

Die Erfindung bezieht sich allgemein auf Hüftimplantate, insbesondere auf Hüftimplantatsysteme mit Augmenten, die an einem Hüftimplantat befestigbar sind und Vorrichtungen, die zur Bearbeitung eines Knochens eines Patienten bei der Implantation derartiger Hüftimplantate verwendet werden können.

Künstliche Hüftgelenke können eine Femurkomponente mit einem in den Oberschenkelknochen des Patienten einzusetzenden Schaft und einer künstlichen Gelenkkugel, sowie eine künstliche Gelenkpfanne, die am Hüftknochen des Patienten angebracht wird, umfassen.

In manchen Fällen, beispielsweise, wenn sich die Hüftgelenksprothese eines Patienten gelockert hat, kann es erforderlich sein, eine Revisionsoperation durchzuführen, bei der die Hüftgelenksprothese ganz oder teilweise ausgetauscht wird. Bei einem teilweisen Austausch der Hüftgelenksprothese kann entweder die künstliche Gelenkpfanne oder die Femurkomponente der Hüftgelenksprothese ausgetauscht werden.

Insbesondere bei Revisionsoperationen, bei denen das ganze künstliche Hüftgelenk ausgetauscht wird, oder bei Operationen, bei denen die künstliche Gelenkpfanne ausgetauscht wird, können aufgrund von Knochendefekten am Hüftknochen des Patienten Schwierigkeiten auftreten. Angesichts dieser Situation wurden spezielle Hüftimplantate zum Befestigen einer künstlichen Gelenkpfanne am Hüftknochen eines Patienten bei einer Revisionsoperation entwickelt. US2005/0107799 A1 offenbart einen Apparat zum Implantieren einer Hüftendoprothese, der eine Probegelenkspfanne, einen Probegelenkskopf mit Mitteln zum Ausrichten der Probegelenkspfanne im Acetabulum und eine Vorrichtung zum Festlegen der ausgerichteten Position der Probegelenkspfanne aufweist.

Ein modulares Hüftimplantat nach dem Stand der Technik, das bei einer Revisionsoperation, bei der die Hüftgelenkspfanne des Patienten ausgetauscht wird, verwendet werden kann, ist in der WO 2007/118708 A2 beschrieben. Das modulare Hüftimplantat umfasst einen Grundkörper zum Befestigen an einem Beckenknochen und eine Pfanne zum Aufnehmen einer Hüftgelenksprothese. Der Grundkörper weist Befestigungsmittel zum Anbringen des Grundkörpers an dem Beckenknochen und einen konkaven Aufnahmebereich für die Pfanne auf. Die Pfanne weist eine zu dem Aufnahmebereich komplementäre konvexe Außenform auf, so dass die den konkaven Aufnahmebereich und die konvexe Außenform bestimmenden Krümmungsradien einander im Wesentlichen entsprechen. Die Pfanne ist in dem Grundkörper befestigbar. Der Grundkörper und/oder die Pfanne umfassen Einstellmittel zum Einstellen der Anordnung der Pfanne bezüglich des Grundkörpers. Dadurch wird ermöglicht, die Inklination und Anteversion durch eine geeignete Anordnung der Pfanne im Grundkörper einzustellen. Um eine sichere Befestigung des Grundkörpers am Hüftknochen auch im Fall von Knochendefekten zu ermöglichen, können am Grundkörper kraniale Laschen mit Bohrungen, in die Knochenschrauben zum Anschrauben des Grundkörpers am Knochen des Patienten eingesetzt werden können, sowie ein kaudaler Befestigungshaken vorgesehen sein.

Während es in manchen Fällen ausreichend sein kann, den Grundkörper des Hüftimplantats am Hüftknochen des Patienten zu befestigen und eine Rekonstruktion der Knochendefekte des Patienten mit Hilfe von natürlichem Knochenmaterial, das beispielsweise durch eine Öffnung im Grundkörper des Hüftimplantats eingebracht werden kann, durchzuführen, kann es in anderen Fällen sinnvoll sein, ein sogenanntes Augment zu verwenden, um Zwischenräume zwischen dem Grundkörper des Hüftimplantats und dem Knochen des Patienten, die durch Knochendefekte bedingt sind, zu füllen. Ein solches Augment kann aus einem Metall mit einer porösen Struktur gebildet sein, um das Verwachsen des Augments mit dem Knochen des Patienten zu begünstigen und es kann mit Hilfe von Schrauben und/oder Knochenzement am Hüftimplantat befestigt werden.

Bei der Verwendung von Augmenten kann es erforderlich sein, den Knochen des Patienten zu bearbeiten, um die Form des Zwischenraums zwischen dem Hüftimplantat und dem Knochen des Patienten zum Unterbringen des Augments im Zwischenraum anzupassen.

Zu diesem Zweck können Knochenfräser verwendet werden, die von einem Arzt freihändig geführt werden. Das freihändige Fräsen ist jedoch aufwändig und relativ ungenau, so dass ein Risiko besteht, dass zu viel Knochenmaterial des Patienten entfernt wird.

Es ist eine Aufgabe der Erfindung, Vorrichtungen und Systeme bereitzustellen, mit denen die oben beschriebenen Nachteile des Stands der Technik ganz oder teilweise vermieden werden können.

Erfindungsgemäß wird die Aufgabe durch eine Vorrichtung zum Anbringen eines Positionierungsmittels an einem Knochen eines Patienten in einer vorgegebenen Position relativ zu einem Hüftimplantat gelöst. Die Vorrichtung umfasst ein Probehüftimplantat und eine Schablone. Das Probehüftimplantat weist eine Auflagefläche zur Auflage auf dem Knochen des Patienten auf. Die Schablone ist an dem Probehüftimplantat befestigbar und weist mindestens eine zylindrische Öffnung mit einer Längsachse auf. Das Probehüftimplantat hat eine solche Form, dass jede Gerade, die durch eine zylindrische Öffnung verläuft und zur Längsachse der jeweiligen Öffnung parallel ist, das Probehüftimplantat nicht schneidet, wenn die Schablone an dem Probehüftimplantat befestigt ist.

Durch die mindestens eine zylindrische Öffnung der Schablone kann ein Positionierungsmittel, beispielsweise eine Knochenschraube, am Knochen des Patienten angebracht werden, wobei die Position des Positionierungsmittels relativ zum Probehüftimplantat zumindest in zwei Achsen, die zur Längsachse der zylindrischen Öffnung der Schablone senkrecht sind, festgelegt ist. Das Positionierungsmittel kann nach Entfernen des Probehüftimplantats und der Schablone vom Knochen zum Führen eines Bearbeitungswerkzeugs, wie beispielsweise eines Knochenfräsers, verwendet werden. Dadurch kann der Knochen des Patienten gezielt und mit relativ hoher Genauigkeit an Stellen bearbeitet werden, die sich relativ zu dem Probehüftimplantat in bestimmten Positionen befinden. Da die Auflagefläche des Probehüftimplantats eine Form haben kann, die im Wesentlichen der Form der Auflagefläche des endgültigen Hüftimplantats entspricht, kann sich das Probehüftimplantat relativ zum Knochen des Patienten im Wesentlichen in der gleichen Position befinden wie das endgültige Hüftimplantat. Dadurch kann der Knochen des Patienten mit relativ hoher Genauigkeit in der Umgebung des endgültigen Hüftimplantats bearbeitet werden kann, insbesondere an einer Stelle, an der ein Augment am Hüftimplantat angebracht wird.

Das Probehüftimplantat umfasst erfindungsgemäß einen Abschnitt mit einer allgemeinen Form einer Kugelkalottenschale und einen Randabschnitt und die Schablone weist auf einer Seite, die dem Probehüftimplantat zugewandt ist, wenn die Schablone an dem Probehüftimplantat befestigt ist, eine Oberfläche mit einem Abschnitt, der eine zu dem Randabschnitt komplementäre Form hat, auf. Dadurch kann eine besonders stabile Auflage der Schablone auf dem Probehüftimplantat erreicht werden.

Das Probehüftimplantat weist erfindunsgemäß einen schlitzförmigen Einschnitt auf, der sich durch den Abschnitt mit der allgemeinen Form einer Kugelkalottenschale und den Randabschnitt erstreckt, wobei für mindestens eine zylindrische Öffnung gilt, dass jede Gerade, die durch die Öffnung verläuft und zur Längsachse der Öffnung parallel ist, durch den schlitzförmigen Einschnitt verläuft, wenn die Schablone an dem Probehüftimplantat befestigt wird. Durch den schlitzförmigen Einschnitt, der am Probehüftimplantat vorgesehen ist, aber am endgültigen Hüftimplantat nicht vorhanden sein muss, kann auf besonders einfache Weise sichergestellt werden, dass sich zwischen dem Knochen des Patienten und der Schablone keine Teile des Probehüftimplantats befinden, die beim Anbringen eines Positionierungsmittels durch eine zylindrische Öffnung der Schablone stören.

In manchen Ausführungsformen weist die Schablone mehrere zylindrische Öffnungen auf, die in einer Reihe angeordnet sind. Dadurch kann unter Verwendung der gleichen Schablone das Positionierungsmittel relativ zu dem Probehüftimplantat an mehreren verschiedenen Positionen positioniert werden. Dadurch können, je nach Bedarf, unterschiedlich große Bereiche des Knochens des Patienten bearbeitet werden, insbesondere in Abhängigkeit von der Größe eines verwendeten Augments.

In manchen Ausführungsformen sind die Längsachsen der zylindrischen Öffnungen der Schablone zueinander im Wesentlichen parallel.

In manchen Ausführungsformen hat die Schablone auf einer Seite, die von dem Probehüftimplantat abgewandt ist, wenn die Schablone an dem Probehüftimplantat befestigt ist, eine abgestufte Oberfläche mit mehreren Stufen, wobei sich durch jede der Stufen eine der mehreren zylinderförmigen Öffnungen erstreckt. Die Stufen können eine Referenz zum Positionieren eines Positionierungsmittels, das durch eine der zylinderförmigen Öffnungen hindurch am Knochen des Patienten angebracht wird, in einer Richtung parallel zur Längsachse der zylindrischen Öffnung dienen. Dadurch kann die Position des Positionierungsmittels relativ zum Probehüftimplantat auch in einer Achse, die zur Längsachse der zylindrischen Öffnung parallel ist, genau eingestellt werden.

In manchen Ausführungsformen hat zumindest ein Teil von jeder der Stufen eine Oberfläche, die zu der Längsachse der zylindrischen Öffnung, die sich durch die jeweilige Stufe erstreckt, senkrecht ist. Dadurch wird eine besonders genaue Positionierung eines Positionierungsmittels, das durch eine der zylinderförmigen Öffnungen hindurch am Knochen des Patienten angebracht wird, entlang der zur Längsrichtung der zylindrischen Öffnung parallelen Achse ermöglicht.

In manchen Ausführungsformen weist das Probehüftimplantat auf einer ersten Seite des schlitzförmigen Einschnitts eine Öffnung im Randabschnitt auf. Die Schablone weist einen zu der Öffnung im Randabschnitt komplementären Vorsprung auf, der in die Öffnung im Randabschnitt eingreift, wenn die Schablone an dem Probehüftimplantat befestigt ist. Auf einer zweiten Seite des schlitzförmigen Einschnitts weist das Probehüftimplantat eine Gewindeöffnung im Randabschnitt mit einem Innengewinde auf. Die Schablone weist eine von der genannten mindestens einen zylindrischen Öffnung separate Öffnung auf, die mit der Gewindeöffnung im Randabschnitt fluchtet, wenn die Schablone an dem Probehüftimplantat befestigt ist. Die Vorrichtung umfasst zusätzlich eine Schraube mit einem zu dem Innengewinde in der Gewindeöffnung im Randabschnitt komplementären Außengewinde zum Verschrauben der Schablone mit dem Probehüftimplantat.

Durch die Gewindeöffnung im Randabschnitt, die mit der Gewindeöffnung im Randabschnitt fluchtende Öffnung in der Schablone und die Schraube kann die Schablone stabil am Probehüftimplantat fixiert werden. Durch die Öffnung im Randabschnitt des Probehüftimplantats auf der der Gewindeöffnung gegenüberliegenden Seite des schlitzförmigen Einschnitts und den zu dieser Öffnung komplementären Vorsprung der Schablone kann ein Verdrehen der Schablone um die Längsachse der Schraube relativ zum Probehüftimplantat verhindert werden.

In manchen Ausführungsformen umfasst die Vorrichtung zusätzlich eine Bohrerführung, die durch die mindestens eine zylindrische Öffnung der Schablone steckbar ist und einen Kanal aufweist, durch den ein Knochenbohrer führbar ist. Mit Hilfe des durch den Kanal der Bohrerführung geführten Bohrers kann der Knochen des Patienten vor dem Anbringen des Positionierungsmittels vorgebohrt werden. Durch die Bohrerführung kann sichergestellt werden, dass der Bohrer parallel zur Längsachse der zylindrischen Öffnung ausgerichtet ist, obwohl der Durchmesser des Bohrers kleiner als der Durchmesser der zylindrischen Öffnung ist.

In manchen Ausführungsformen umfasst die Vorrichtung zusätzlich ein Positionierungsmittel. Das Positionierungsmittel weist einen zylindrischen Führungsabschnitt mit einer Längsachse auf, der durch die mindestens eine zylindrische Öffnung der Schablone führbar ist. Der Führungsabschnitt ist dabei relativ zur Schablone entlang der Längsachse der zylindrischen Öffnung beweglich und die Längsachse des Führungsabschnitts ist entlang der Längsachse der zylindrischen Öffnung ausgerichtet. Durch Verwendung eines derart ausgebildeten Positionierungsmittels kann eine besonders genaue Positionierung des Positionierungsmittels im Knochen des Patienten erreicht werden.

In manchen Ausführungsformen umfasst die Vorrichtung zusätzlich ein Werkzeug zum Befestigen des Positionierungsmittels am Knochen des Patienten durch eine zylindrische Öffnung der Schablone hindurch. Das Werkzeug umfasst einen Anschlag zum Festlegen eines Abstands zwischen einem distalen Ende des am Knochen des Patienten befestigten Positionierungsmittels und dem vom Probeimplantat abgewandten Ende der zylindrischen Öffnung der Schablone, durch die hindurch das Positionierungsmittel am Knochen des Patienten befestigt wird. Dadurch kann das Positionierungsmittel auf eine besonders einfache Weise entlang der zur Längsachse der zylindrischen Öffnung parallelen Achse positioniert werden.

In manchen Ausführungsformen umfasst das Positionierungsmittel eine Knochenschraube. Das Werkzeug zum Befestigen des Positionierungsmittel am Knochen des Patienten umfasst einen Schraubendreher und der Anschlag des Werkzeugs zum Befestigen des Positionierungsmittels wird durch einen Abschnitt eines Schafts des Schraubendrehers, der einen größeren Durchmesser aufweist als die mindestens eine zylindrische Öffnung der Schablone, bereitgestellt. In solchen Ausführungsformen kann die Knochenschraube mit Hilfe des Schraubendrehers in den Knochen des Patienten eingeschraubt werden. Sobald der Abschnitt des Schraubendrehers mit dem größeren Durchmesser am Rand der zylindrischen Öffnung der Schablone anstößt, kann sich der Schraubendreher dem Knochen des Patienten nicht mehr weiter annähern, wodurch die Tiefe, bis zu der die Knochenschraube in den Knochen eingeschraubt werden kann, begrenzt wird.

Eine erfindungsgemäße Vorrichtung zum Bearbeiten eines Knochens eines Patienten bei einer Implantation eines Hüftimplantats umfasst eine Vorrichtung zum Anbringen eines Positionierungsmittels an einem Knochen eines Patienten mit einigen oder allen der oben beschriebenen Merkmale und mindestens einen Knochenfräser. Der Knochenfräser weist einen Aufnahmeabschnitt für den Führungsabschnitt des Positionierungsmittels auf. Der Führungsabschnitt ist in den Aufnahmeabschnitt einsetzbar. Der Aufnahmeabschnitt ist so ausgebildet, dass eine Drehachse des Knochenfräsers entlang der Längsachse des Führungsabschnitts ausgerichtet ist und der Knochenfräser um die Längsachse des Führungsabschnitts drehbar und entlang der Längsachse des Führungsabschnitts verschiebbar ist, wenn der Führungsabschnitt in den Aufnahmeabschnitt des Knochenfräsers eingesetzt ist.

Durch das Einsetzen des Führungsabschnitts des Positionierungsmittels in den Aufnahmeabschnitt des Knochenfräsers kann eine Position des Knochenfräsers in zwei Achsen, die zur Längsrichtung des Führungsabschnitts des Positionierungsmittels senkrecht sind, im Wesentlichen festgelegt werden. Da die Längsachse des Führungsabschnitts des Positionierungsmittels im Wesentlichen entlang der Längsachse der zylindrischen Öffnung der Schablone, durch die hindurch das Positionierungsmittel am Knochen des Patienten angebracht wurde, ausgerichtet ist, kann so die Position des Knochenfräsers relativ zum Probehüftimplantat und damit auch relativ zum endgültigen Hüftimplantat in zwei Achsen festgelegt werden. Während des Fräsens des Knochens des Patienten kann der Knochenfräser entlang der Längsachse des Führungsabschnitts auf dem Knochen zu bewegt werden und dabei rotieren, wobei der Knochen des Patienten abgetragen wird.

In manchen Ausführungsformen umfasst der Aufnahmeabschnitt des Knochenfräsers einen Anschlag für das distale Ende des Positionierungsmittels. Dadurch kann die Tiefe, bis der der Knochen des Patienten abgefräst werden kann, begrenzt werden. Da, wie oben ausgeführt, die Position des distalen Endes des Positionierungsmittels entlang der Längsrichtung der zylindrischen Öffnung, durch die das Positionierungsmittel am Knochen des Patienten angebracht wird, relativ genau festgelegt werden kann, ist somit die Lage des vom Knochen des Patienten abgetragenen Bereichs in drei Achsen festgelegt.

Ein erfindungsgemäßes Hüftimplantatsystem umfasst eine Vorrichtung zum Bearbeiten eines Knochens eines Patienten mit einigen oder allen der oben beschriebenen Merkmale, ein Hüftimplantat und mindestens ein Augment, das an dem Hüftimplantat befestigbar ist. Das Hüftimplantat dient zur Aufnahme einer künstlichen Gelenkpfanne und umfasst eine Auflagefläche zur Auflage auf dem Knochen des Patienten, die eine Form hat, die der Form der Auflagefläche des Probehüftimplantats entspricht. Das Augment weist auf einer Seite, die von dem Hüftimplantat abgewandt ist, wenn das Augment an dem Hüftimplantat befestigt ist, eine Oberfläche mit einer Form, die zu einer Form eines Bearbeitungsabschnitts eines Knochenfräsers korrespondiert, auf.

Da die Form der Auflagefläche des Hüftimplantats der Form der Auflagefläche des Probehüftimplantats entspricht, kann das endgültige Hüftimplantat am Knochen des Patienten im Wesentlichen an der gleichen Stelle und in der gleichen Orientierung angebracht werden wie zuvor das Probehüftimplantat. Durch die entsprechenden Formen der vom Hüftimplantat abgewandten Seite des Augments und des Bearbeitungsabschnitts des verwendeten Knochenfräsers kann erreicht werden, dass die Form des Abschnitts des Knochens, der das Augment kontaktiert, eine Form hat, die zur Form des den Knochen berührenden Teils des Augments korrespondiert. Dadurch wird ein relativ schnelles Anwachsen des Augments am Knochen des Patienten, durch das die Stabilität des Hüftimplantats verbessert wird, erreicht.

In manchen Ausführungsformen weist die Schablone mehrere zylindrische Öffnungen auf. Das Hüftimplantatsystem umfasst mehrere verschieden große Augmente. Jeder Knochenfräser weist einen Bearbeitungsabschnitt mit einer allgemein sphärischen Form auf. Jedem Augment ist eine der zylindrischen Öffnungen der Schablone und ein Knochenfräser zugeordnet. Jedes Augment weist auf der Seite, die von dem Hüftimplantat abgewandt ist, wenn das Augment an dem Hüftimplantat befestigt ist, eine allgemein sphärische Oberfläche auf, wobei ein Radius der allgemein sphärischen Oberfläche einem Radius des allgemein sphärischen Bearbeitungsabschnitts des Knochenfräsers, der dem Augment zugeordnet ist, entspricht. Dadurch wird ein modulares System bereitgestellt, das eine flexible Anpassung des Hüftimplantatsystems an den Zustand des Knochens des Patienten und eine entsprechende Bearbeitung des Knochens des Patienten beim Implantieren des Hüftimplantats ermöglicht.

Im Folgenden werden Ausführungsformen der Erfindung mit Bezug auf die Figuren beschrieben. Es zeigen:
- Fig. 1: zeigt eine schematische Ansicht einer Schablone;
- Fig. 2: zeigt eine schematische Ansicht eines Probehüftimplantats;
- Fig. 3: zeigt eine schematische Ansicht der in Fig. 1 dargestellten Schablone, in der die in der Ansicht der Fig. 1 vom Betrachter abgewandte Seite der Schablone zu sehen ist;
- Fig. 4: zeigt eine schematische Ansicht einer erfindungsgemäßen Bohrerführung;
- Fig. 5: zeigt eine schematische Ansicht eines Knochenbohrers;
- Fig. 6: zeigt eine schematische Ansicht eines Positionierungsmittels;
- Fig. 7: zeigt eine schematische Ansicht eines Werkzeugs zum Befestigen des in Fig. 6 dargestellten Positionierungsmittels an einem Knochen;
- Fig. 8: zeigt eine schematische Ansicht eines Knochenfräsers;
- Fig. 9A: zeigt eine schematische Ansicht eines Hüftimplantats mit einem daran befestigten Augment;
- Fig. 9B: zeigt eine schematische Ansicht des in Fig. 9A dargestellten Augments aus einer anderen Blickrichtung;
- Fig. 10A-10G: zeigen schematische Ansichten eines Hüftknochens während der Bearbeitung mit Hilfe einer erfindungsgemäßen Vorrichtung; und
- Fig. 11: zeigt eine schematische Ansicht eines Messgeräts zum Bestimmen der Länge des zu verwendenden Positionierungsmittels.

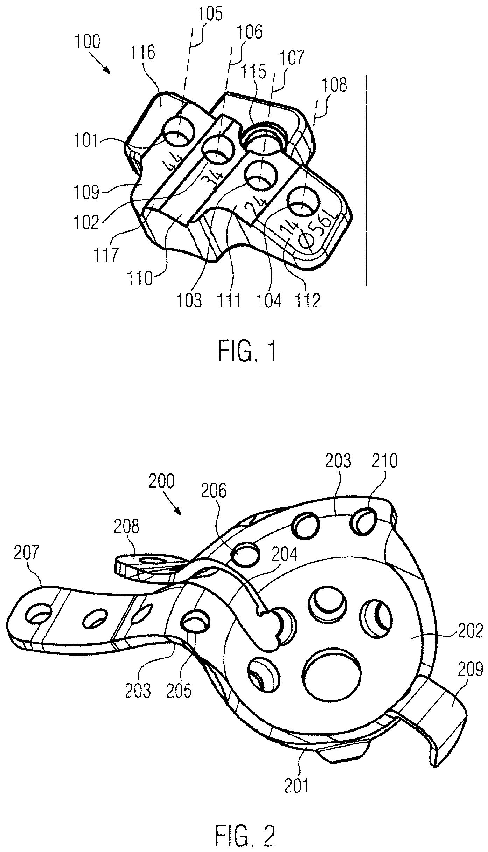

Fig. 1 zeigt eine schematische Ansicht einer Schablone 100, die Teil einer erfindungsgemäßen Vorrichtung sein kann.

Die Schablone 100 weist mehrere zylindrische Öffnungen 101, 102, 103, 104 auf, die sich durch die Schablone 100 hindurch erstrecken. In Fig. 1 ist eine Ausführungsform der Schablone 100 dargestellt, in der die Schablone 100 vier zylindrische Öffnungen 101, 102, 103, 104 aufweist. In anderen Ausführungsformen kann eine größere Anzahl von zylindrischen Öffnungen oder eine kleinere Anzahl von zylindrischen Öffnungen, beispielsweise eine, zwei oder drei zylindrische Öffnungen, vorhanden sein.

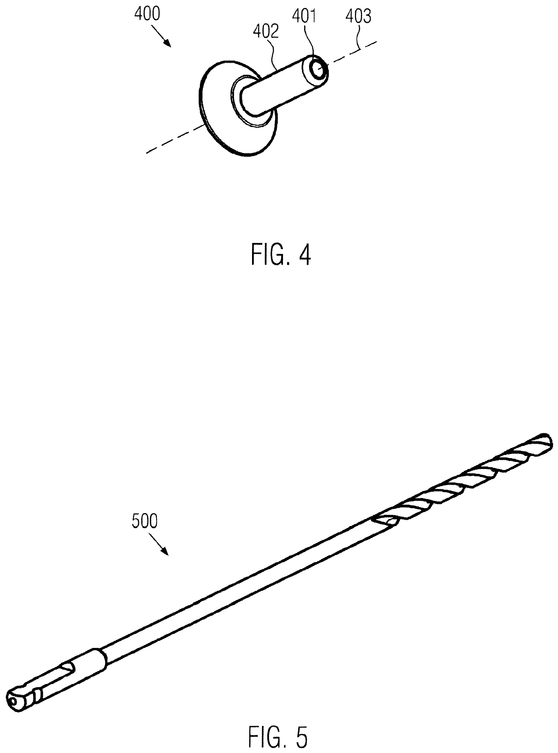

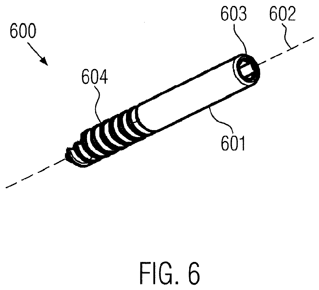

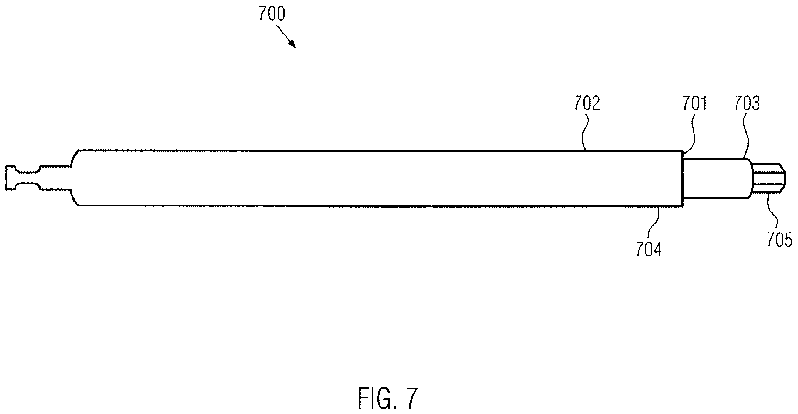

Längsachsen der zylindrischen Öffnungen 101, 102, 103, 104 sind in Fig. 1 durch gestrichelte Linien 105, 106, 107, 108 dargestellt. In manchen Ausführungsformen können die Längsachsen 105, 106, 107, 108 der zylindrischen Öffnungen 101, 102, 103, 104 zueinander im Wesentlichen parallel sein. In anderen Ausführungsformen können die Längsachsen 105, 106, 107, 108 der zylindrischen Öffnungen 101, 102, 103, 104 zueinander geneigt sein. Die zylindrischen Öffnungen 101, 102, 103, 104 können im Wesentlichen den gleichen Durchmesser haben, so dass Elemente, die bei der Benutzung der erfindungsgemäßen Vorrichtung mit den Öffnungen 101, 102, 103, 104 zusammenwirken, wie beispielsweise die in Fig. 4 dargestellte Bohrerführung 400, das in Fig. 6 dargestellte Positionierungsmittel 600 und das in Fig. 7 dargestellte Werkzeug 700 mit allen zylindrischen Öffnungen 101, 102, 103, 104 der Schablone 100 gleichermaßen verwendet werden können.

Die zylindrischen Öffnungen 101, 102, 103, 104 können in einer Reihe angeordnet sein, wobei die Längsachsen 105, 106, 107, 108 der zylindrischen Öffnungen 101, 102, 103, 104 im Wesentlichen in einer Ebene liegen.

Die Schablone 100 kann auf einer Seite eine abgestufte Oberfläche haben, die mehrere Stufen 109, 110, 111, 112 aufweist. Die zylindrische Öffnung 101 erstreckt sich durch die Stufe 109, die zylindrische Öffnung 102 erstreckt sich durch die Stufe 110, die zylindrische Öffnung 103 erstreckt durch die Stufe 111 und die zylindrische Öffnung 104 erstreckt sich durch die Stufe 112. Zumindest ein Teil von jeder der Stufen 109, 110, 111, 112 kann eine Oberfläche haben, die zu der Längsachse der zylindrischen Öffnung, die sich durch die jeweilige Stufe erstreckt, senkrecht ist. In manchen Ausführungsformen können an einigen oder allen der Stufen 109, 110, 111, 112 Abschrägungen vorhanden sein. In der in Fig. 1 dargestellten Ausführungsform weist beispielsweise die Stufe 109 eine Abschrägung 116 auf und die Stufe 110 weist eine Abschrägung 117 auf, während an den Stufen 111, 112 keine Abschrägungen vorhanden sind.

Fig. 2 zeigt eine schematische Ansicht eines Probehüftimplantats 200, das zusammen mit der in Fig. 1 dargestellten Schablone 100 verwendet werden kann, und in Kombination mit der Schablone 100 eine erfindungsgemäße Vorrichtung bildet. Das Probehüftimplantat 200 weist eine Auflagefläche 201 zur Auflage auf einem Knochen eines Patienten auf. In der Ansicht der Fig. 2 befinden sich große Teile der Auflagefläche 201 auf der vom Betrachter abgewandten Seite des Probehüftimplantats 200 und sind deshalb verdeckt. In Ausführungsformen können Teile der Auflagefläche 201 eine allgemein sphärische Form haben, wobei unter einer allgemein sphärischen Form eine Form verstanden werden kann, die im Wesentlichen einer Kugeloberfläche oder einem Teil einer Kugeloberfläche entspricht, wobei Abweichungen von einer idealen Kugelform wie beispielsweise eine gewisse Rauigkeit, Öffnungen und/oder Vertiefungen vorhanden sein können.

Das Probehüftimplantat 200 umfasst einen Abschnitt 202 mit einer allgemeinen Form einer Kugelkalottenschale, auf dessen Außenseite sich der Teil der Auflagefläche 201 mit der allgemein sphärischen Form befindet. Außerdem umfasst das Probehüftimplantat 200 einen Randabschnitt 203, an dem Laschen 207, 208 angebracht sein können. Auf der von den Laschen 207, 208 abgewandten Seite des Probehüftimplantats 200 kann ein Haken 209 angebracht sein, der in den Hüftknochen des Patienten eingehängt werden kann. In dem Abschnitt 202 mit der allgemeinen Form einer Kugelkalottenschale, dem Randabschnitt 203 und den Laschen 207, 208 können Bohrungen vorhanden sein, von denen eine beispielhaft mit dem Bezugszeichen 210 versehen ist. Durch die Bohrungen 210 können Schrauben geführt werden, mit denen das Probehüftimplantat 200 am Hüftknochen des Patienten fixiert werden kann.

Die oben beschriebenen Merkmale des Probehüftimplantats 200 können im Wesentlichen Merkmalen eines endgültigen Hüftimplantats, das einem Patienten dauerhaft implantiert wird, entsprechen.

Zusätzlich zu den oben beschriebenen Merkmalen kann das Probehüftimplantat 200 einen schlitzförmigen Einschnitt 204 aufweisen, der sich durch den Randabschnitt 203 und in den Abschnitt 202 mit der allgemeinen Form einer Kugelkalottenschale hinein erstreckt. In Ausführungsformen, in denen das Probehüftimplantat 200 Laschen 207, 208 aufweist, kann der schlitzförmige Einschnitt 204 in den Zwischenraum zwischen den Laschen 207, 208 münden. An dem endgültigen Hüftimplantat, das dem Patienten implantiert wird, muss kein dem schlitzförmigen Einschnitt 204 entsprechendes Element vorhanden sein, wodurch das endgültige Hüftimplantat im Vergleich zu dem Probehüftimplantat 200 stabiler sein kann. Das endgültige Hüftimplantat kann noch weitere Merkmale umfassen, die sich von denen des Probehüftimplantats 200 unterscheiden. In manchen Ausführungsformen kann das endgültige Hüftimplantat ein Hüftimplantat von dem in der WO 2007/118708 A2 beschriebenen Typ sein.

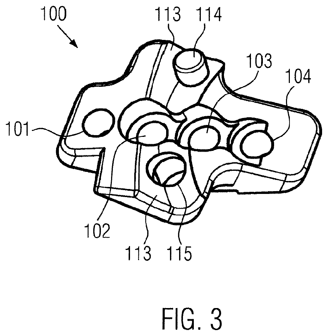

Fig. 3 zeigt eine Ansicht der in der Fig. 1 dargestellten Schablone 100 aus einer anderen Blickrichtung, aus der die in der Darstellung der Fig. 1 vom Betrachter abgewandte Seite des Implantats 100 sichtbar ist. Auf dieser Seite hat das Implantat 100 eine Oberfläche mit einem Abschnitt 113, der eine zu dem Randabschnitt 203 des Probehüftimplantats 200 komplementäre Form hat und auf dem Randabschnitt 203 des Probehüftimplantats 200 aufliegen kann. Dabei ist die in der Darstellung der Fig. 3 dem Betrachter zugewandte Seite, auf der sich der Abschnitt 113 befindet, dem Probehüftimplantat 200 zugewandt und die in der Fig. 1 sichtbare Seite der Schablone 100, auf der sich die Stufen 109, 110, 111, 112 befinden, ist vom Probehüftimplantat 200 abgewandt.

Die Schablone 100 ist dafür ausgelegt, in der oben beschriebenen Position an dem Probehüftimplantat 200 befestigt zu werden. Zu diesem Zweck kann die Schablone 100 eine von den zylindrischen Öffnungen 101, 102, 103, 104 separate Öffnung 115 aufweisen, die mit einer Gewindeöffnung 206 im Randabschnitt 203 des Probehüftimplantats 200 fluchtet, wenn der Abschnitt 113 der Oberfläche der Schablone 100 auf dem Randabschnitt 203 des Probehüftimplantats 200 aufliegt. Durch die Öffnung 115 der Schablone 100 kann dann eine Schraube mit einem zu dem Innengewinde der Gewindeöffnung 206 im Randabschnitt 203 des Probehüftimplantats 200 komplementären Außengewinde geführt und in die Gewindeöffnung 206 eingeschraubt werden, wodurch die Schablone 100 lösbar am Probehüftimplantat 200 fixiert wird.

Auf der der Gewindeöffnung 206 gegenüberliegenden Seite des schlitzförmigen Einschnitts 204 kann sich im Randabschnitt 203 des Probehüftimplantats 200 eine Öffnung 205 befinden. An der Schablone 100 kann sich ein Vorsprung 114 befinden, der zu der Öffnung 205 im Randabschnitt 203 des Probehüftimplantats 200 komplementär ist und in die Öffnung 205 eingreift, wenn der Abschnitt 113 der Oberfläche der Schablone auf dem Randabschnitt 203 des Probehüftimplantats 200 aufliegt. Durch den Vorsprung 114 und die Öffnung 205 kann eine Rotation der Schablone 100 relativ zu dem Probehüftimplantat 200 verhindert werden.

Wenn die Schablone 100, wie oben beschrieben, an dem Probehüftimplantat 200 befestigt ist, sind die zylindrischen Öffnungen 102, 103, 104 der Schablone 100 gegenüber dem schlitzförmigen Einschnitt 204 des Probehüftimplantats angeordnet, während die zylindrische Öffnung 101 der Schablone 100 einem Zwischenraum zwischen den Laschen 207, 208 des Probehüftimplantats 200 gegenüber angeordnet ist. Die Breiten des schlitzförmigen Einschnitts 204 und des sich an den schlitzförmigen Einschnitt 204 anschließenden Zwischenraums zwischen den Laschen 207, 208 können größer als der Durchmesser der zylindrischen Öffnungen 101, 102, 103, 104 sein, so dass jede Gerade, die durch eine der zylindrischen Öffnungen 101, 102, 103, 104 verläuft und zu der Längsachse 105, 106, 107 bzw. 108 der jeweiligen zylindrischen Öffnung parallel ist, durch den schlitzförmigen Einschnitt 204 oder den Zwischenraum zwischen den Laschen 207, 208 verläuft, ohne das Probehüftimplantat 200 zu schneiden. Infolgedessen kann durch jede der zylindrischen Öffnungen 101, 102, 103, 104 und den Zwischenraum zwischen den Laschen 207, 208 bzw. den schlitzförmigen Einschnitt 204 des Probehüftimplantats 200 ein zylindrisches Objekt mit einem dem Durchmesser der zylindrischen Öffnungen 101, 102, 103, 104 entsprechenden Durchmesser geführt werden, ohne an dem Probehüftimplantat 200 anzustoßen. Insbesondere gilt dies für das unten mit Bezug auf Fig. 6 genauer beschriebene Positionierungsmittel 600, das einen zylindrischen Führungsabschnitt 601 umfasst.

Fig. 4 zeigt eine Bohrerführung 400. Die Bohrerführung umfasst einen zylindrischen Abschnitt 402 mit einem Durchmesser, der dem Durchmesser der zylindrischen Öffnungen 101, 102, 103, 104 der Schablone 100 entspricht. Somit kann der zylindrische Abschnitt 402 der Bohrerführung 400 durch jede der zylindrischen Öffnungen 101, 102, 103, 104 der Schablone 100 gesteckt werden, wobei eine Längsachse 403 des zylindrischen Abschnitts 402 der Bohrerführung 400 an der Längsachse der jeweiligen zylindrischen Öffnung der Schablone 100 ausgerichtet ist. Die Bohrerführung 400 weist einen Kanal 401 auf, der sich entlang der Längsachse 403 des zylindrischen Abschnitts 402 erstreckt. Durch den Kanal 401 kann ein in Fig. 5 dargestellter Knochenbohrer 500 geführt werden. Dadurch kann der Knochenbohrer 500 entlang der Längsachse 403 des Kanals 401 der Bohrerführung und damit auch entlang der Längsachse der zylindrischen Öffnung der Schablone 100, in die die Bohrerführung 400 eingesetzt ist, ausgerichtet werden. Die Bohrerführung 400 und der Knochenbohrer 500 können zum Vorbohren eines Knochens eines Patienten beim Anbringen eines Positionierungsmittels 600 verwendet werden, das im Folgenden mit Bezug auf Fig. 6 beschrieben wird.

Das Positionierungsmittel 600 ist eine Knochenschraube mit einem Gewinde 604 und einem zylindrischen Führungsabschnitt 601. Ein Durchmesser des zylindrischen Führungsabschnitts 601 kann einem Durchmesser der zylindrischen Öffnungen 101, 102, 103, 104 der Schablone 100 entsprechen, und ein Außendurchmesser des Gewindes 604 kann kleiner oder gleich dem Durchmesser des Führungsabschnitts 601 sein, so dass das Positionierungsmittel 600 durch jede der zylindrischen Öffnungen 101, 102, 103, 104 geführt werden kann. Während sich der Führungsabschnitt 601 in einer der zylindrischen Öffnungen 101, 102, 103, 104 der Schablone 100 befindet, ist die Längsachse 602 des zylindrischen Führungsabschnitts 601 an der Längsachse der jeweiligen zylindrischen Öffnung ausgerichtet. Das Positionierungsmittel 600 kann dabei entlang der Längsachse der zylindrischen Öffnung bewegt und um die Längsachse der zylindrischen Öffnung gedreht werden. Die Orientierung der Längsachse 602 des Positionierungsmittels 600 und die Position des Führungsmittels 600 in zwei Achsen, die zu der Längsachse der zylindrischen Öffnung, in der sich der Führungsabschnitt 601 befindet, senkrecht sind, sind jedoch relativ zu der Schablone 100 festgelegt. Wenn die Schablone 100 an dem Probehüftimplantat 200 befestigt ist, ist dadurch auch die Position des Positionierungsmittels 600 relativ zum Probehüftimplantat 200 in zwei Achsen festgelegt.

An einem distalen Ende 603 des Positionierungsmittels 600, das sich auf einer vom Gewinde 604 abgewandten Seite des Führungsabschnitts 601 des Positionierungsmittels 600 befindet, ist ein Mitnahmeprofil, beispielsweise ein Innensechskant, vorgesehen.

Fig. 7 zeigt eine Ansicht eines Werkzeugs 700, das verwendet werden kann, um das Gewinde 604 des Positionierungsmittels 600 in den Hüftknochen eines Patienten einzuschrauben, während der Führungsabschnitt 601 des Positionierungsmittels 600 durch eine der zylindrischen Öffnungen 101, 102, 103, 104 der Schablone 100 geführt wird. Das Werkzeug 700 ist ein Schraubendreher mit einem Schaft 704 und einer Klinge 705, die eine zu dem Mitnahmeprofil am distalen Ende 603 des Positionierungsmittels 600 korrespondierende Form hat. An die Klinge 705 schließt sich ein Abschnitt 703 des Schafts 704 an, der eine zylindrische Form und einen Durchmesser, der dem Durchmesser der zylindrischen Öffnungen 101, 102, 103, 104 der Schablone 100 entspricht, hat. Der Schaft 704 des Werkzeugs 700 umfasst außerdem einen Abschnitt 702, der einen größeren Durchmesser aufweist als die zylindrischen Öffnungen 101, 102, 103, 104 und einen Anschlag 701 des Werkzeugs 700 bereitstellt.

Wenn das Positionierungsmittel 600 durch eine der Öffnungen 101, 102, 103, 104 der Schablone 100 in den Knochen des Patienten eingeschraubt wird, wird zunächst der Abschnitt 703 des Schafts 704 des Werkzeugs 700 in die verwendete Öffnung eingeführt, bis schließlich der Anschlag 701 an derjenigen der Stufen 109, 110, 111, 112 der Schablone 100 anstößt, in der sich die Öffnung befindet. Dadurch wird die Tiefe, bis zu der das Positionierungsmittel 600 in den Knochen des Patienten eingeschraubt wird und somit die Position des distalen Endes 603 des Positionierungsmittels relativ zur Schablone 100 in der zu der Längsachse der verwendeten Öffnung der Schablone parallelen Achse festgelegt. Somit kann durch Anbringen des Positionierungsmittels 600 am Knochen des Patienten durch eine der Öffnungen 101, 102, 103, 104 der Schablone 100 mit Hilfe des Werkzeugs 700 die Position des distalen Endes 603 des am Knochen des Patienten befestigen Positionierungsmittels relativ zu dem Probehüftimplantat 200, an dem die Schablone 100 befestigt ist, in drei Achsen festgelegt werden.

Wenn die Schablone 100, wie in den Fig. 1 und 3 dargestellt, mehrere zylindrische Öffnungen 101, 102, 103, 104 aufweist, gibt es mehrere Möglichkeiten, das Positionierungsmittel 600 relativ zu dem Probehüftimplantat 200 in einer definierten Position am Knochen des Patienten anzubringen.

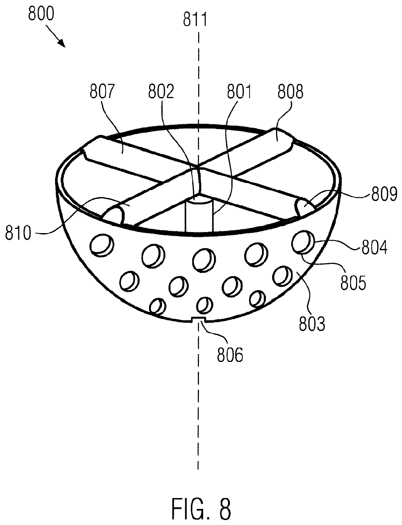

Das wie oben beschrieben in einer definierten Position relativ zum Probehüftimplantat 200 am Knochen des Patienten angebrachte Positionierungsmittel 600 kann in Ausführungsformen der Erfindung dazu verwendet werden, einen Knochenfräser 800, der in Fig. 8 dargestellt ist, zu führen.

Der Knochenfräser 800 umfasst einen Bearbeitungsabschnitt 803. Der Bearbeitungsabschnitt 803 kann eine allgemein sphärische Form haben. Beispielsweise kann der Bearbeitungsabschnitt 803, wie in Fig. 8 dargestellt, im Wesentlichen eine Halbkugelform haben. Der Bearbeitungsabschnitt 803 kann mehrere Schneiden umfassen, von denen eine beispielhaft durch das Bezugszeichen 804 bezeichnet ist. Neben den Schneiden können sich Öffnungen befinden. In Fig. 8 ist die Öffnung, die sich neben der Schneide 804 befindet, beispielhaft durch das Bezugszeichen 805 bezeichnet. Durch die Öffnungen können Knochenspäne, die durch die Schneiden abgetragen werden, ins Innere des Bearbeitungsabschnitts 803 eintreten, so dass sie nach Abschluss der Bearbeitung des Knochens zusammen mit dem Knochenfräser 800 entfernt werden können.

Der Knochenfräser 800 umfasst einen Aufnahmeabschnitt 801 für den Führungsabschnitt 601 des Positionierungsmittels 600. Der Aufnahmeabschnitt 801 kann eine hohlzylindrische Form haben und sich von einer Öffnung 806 am Pol des Bearbeitungsabschnitts 803 in einer Richtung zum Mittelpunkt des Bearbeitungsabschnitts 803 erstrecken. An einem von der Öffnung 806 abgewandten Ende des Aufnahmeabschnitts 801 befindet sich ein Anschlag 802 für das distale Ende 603 des Positionierungsmittels 600. Der Knochenfräser 800 kann außerdem Verstrebungen 807, 808, 809, 810 umfassen, an denen der Knochenfräser 800 von einem Werkzeug zum Rotieren des Knochenfräsers 800 erfasst werden kann. Der Knochenfräser 800 rotiert dabei um eine Längsachse 811 des Aufnahmeabschnitts 801.

Ein Innendurchmesser des Aufnahmeabschnitts 801 des Knochenfräsers kann einem Durchmesser des Führungsabschnitts 601 des Positionierungsmittels 600 entsprechen. Der Führungsabschnitt 601 des am Knochen des Patienten angebrachten Positionierungsmittels 600 kann in den Aufnahmeabschnitt 801 des Knochenfräsers 800 eingesetzt werden und der Knochen des Patienten kann durch Rotieren des Knochenfräsers 800 um die Längsachse 811 des Aufnahmeabschnitts 801 bearbeitet werden. Während der Bearbeitung kann der Knochenfräser 800 auf den Knochen zu bewegt werden, wobei der Führungsabschnitt 601 des Positionierungsmittels weiter in den Aufnahmeabschnitt 801 des Knochenfräsers 800 eindringt, bis das distale Ende 603 des Positionierungsmittels 600 am Anschlag 802 anstößt. Dadurch wird die Bewegung des Knochenfräsers 800 auf den Knochen des Patienten zu und damit das Entfernen von Knochenmaterial durch den Knochenfräser 800 im Wesentlichen beendet.

Durch Verwenden des Knochenfräsers 800 in Verbindung mit dem Positionierungsmittel 600 kann somit Knochenmaterial des Patienten aus einem im Wesentlichen sphärischen Bereich entfernt werden, der durch die Größe des Bearbeitungsabschnitts 803, die Anordnung des Anschlags 802 relativ zum Bearbeitungsabschnitt 803 sowie die Position des distalen Endes 603 des Positionierungsmittels 600 festgelegt ist. Da, wie oben ausgeführt, die Position des distalen Endes 603 des Positionierungsmittels 600 durch Anbringen des Positionierungsmittels 600 am Knochen des Patienten mit Hilfe der am Probehüftimplantat 200 befestigten Schablone 100 und des Werkzeugs 700 relativ zum Probehüftimplantat 200 festgelegt werden kann, kann durch den oben beschriebenen Fräsvorgang Knochenmaterial des Patienten aus einem Bereich, der sich relativ zu dem Probehüftimplantat 200 in einer definierten Position befindet, entfernt werden.

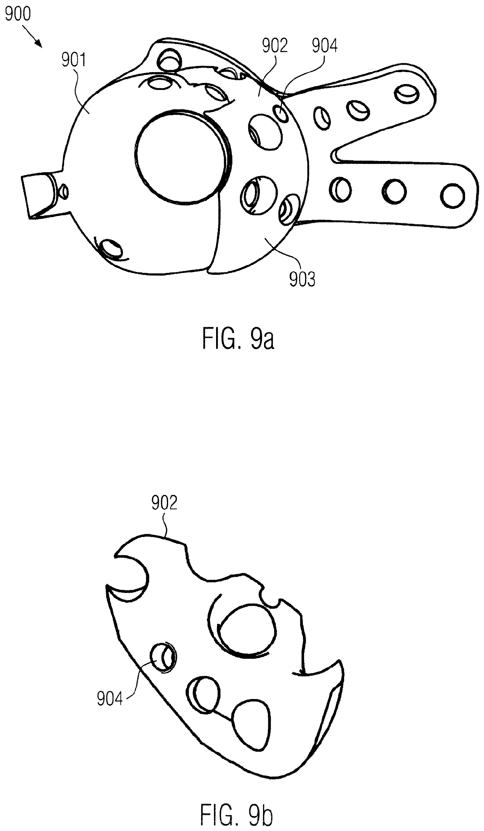

Fig. 9A zeigt eine Ansicht eines Hüftimplantats 900, an dem mit Hilfe einer Schraube 904 ein Augment 902 befestigt ist. Das Hüftimplantat 900 ist zur Aufnahme einer künstlichen Gelenkpfanne eines Patienten geeignet und weist eine Auflagefläche 901 zur Auflage auf einem Knochen des Patienten auf. Die Form der Auflagefläche 901 entspricht der Form der Auflagefläche 201 des Probehüftimplantats 200, so dass das Hüftimplantat 900 nach dem Entfernen des Probehüftimplantats 200 am Knochen des Patienten im Wesentlichen in der gleichen Position befestigt werden kann wie zuvor das Probehüftimplantat 200. Weitere Merkmale des Hüftimplantats 900 können denen des in der WO 2007/118708 A2 beschriebenen Hüftimplantats entsprechen.

Das Augment 902 kann in manchen Ausführungsformen durch ein Sinterverfahren aus einem Metallpulver hergestellt werden, wodurch es eine poröse Struktur erhält, die ein Einwachsen des Knochens des Patienten in das Augment 902 begünstigt. Das Metallpulver kann aus im Wesentlichen reinem Titan oder einer Titanlegierung bestehen.

Auf einer Seite, die vom Hüftimplantat 900 abgewandt ist, wenn das Augment 902 an dem Hüftimplantat 900 befestigt ist, und die in der Ansicht der Fig. 9A dem Betrachter zugewandt ist, kann das Augment 902 eine Oberfläche 903 mit einer Form, die zu einer Form des Bearbeitungsabschnitts 803 des Knochenfräsers 800 korrespondiert, aufweisen. Insbesondere kann die Oberfläche 903 des Augments 902 auf der vom Hüftimplantat 900 abgewandten Seite eine allgemein sphärische Form haben, wobei die Radien der allgemein sphärischen Oberfläche 903 des Augments 902 und des Bearbeitungsabschnitts 803 des Knochenfräsers 800 miteinander korrespondieren können. Beispielsweise kann der Radius der allgemein sphärischen Oberfläche 903 des Augments 902 etwas kleiner als oder ungefähr gleich dem Radius des Bearbeitungsabschnitts 803 des Knochenfräsers 800 sein.

Durch Bearbeiten des Knochens des Patienten mit Hilfe der oben mit Bezug auf die Fig. 1 bis 8 beschriebenen Vorrichtung kann der Knochen des Patienten so bearbeitet werden, dass die allgemein sphärische Oberfläche 903 des Augments 902 nach dem Befestigen des Hüftimplantats 900 am Knochen des Patienten am Knochen anliegt oder sich in unmittelbarer Nähe des Knochens befindet. Dadurch kann ein unnötiges Entfernen von Knochenmaterial vermieden und ein schnelles Verwachsen des Augments 902 mit dem Knochen begünstigt werden.

Ein erfindungsgemäßes Hüftimplantatsystem kann mehrere Hüftimplantate ähnlich dem in Fig. 9A dargestellten Hüftimplantat 900 umfassen. Es können Hüftimplantate unterschiedlicher Größe vorgesehen sein, um eine Anpassung an die individuelle Anatomie eines Patienten zu ermöglichen. Beispielsweise kann das Hüftimplantatsystem Hüftimplantate, die Abschnitte mit der allgemeinen Form einer Kugelkalottenschale unterschiedlichen Durchmessers umfassen, enthalten. Beispielsweise können Durchmesser des Abschnitts mit der allgemeinen Form einer Kugelkalottenschale von ungefähr 48 mm, ungefähr 52 mm, ungefähr 56 mm, ungefähr 60 mm und ungefähr 64 mm vorgesehen sein. Außerdem können in jeder Größe unterschiedliche Hüftimplantate für einen Ersatz des linken Hüftgelenks und des rechten Hüftgelenks vorgesehen sein, wobei die Hüftimplantate für den Ersatz des linken Hüftgelenks zu denen für den Ersatz des rechten Hüftgelenks spiegelsymmetrisch sind. Zu jedem der verschiedenen Hüftimplantate kann ein entsprechendes Probeimplantat vorgesehen sein, das Merkmale entsprechend denen des oben mit Bezug auf Fig. 2 beschriebenen Probeimplantats 200 hat. Mit Hilfe der Probeimplantate kann ausprobiert werden, welches der Hüftimplantate am besten zur individuellen Anatomie des Patienten passt.

Für jedes der verschiedenen Hüftimplantate können Augmente unterschiedlicher Größen vorhanden sein. Beispielsweise können für jedes Hüftimplantat vier verschiedene Augmente vorhanden sein.

Für jedes der Probehüftimplantate kann eine eigene Schablone mit Merkmalen entsprechend der oben mit Bezug auf die Fig. 1 und 3 beschriebenen Schablone 100 vorgesehen sein, wobei die Schablonen entsprechend der unterschiedlichen Dimensionen der Probehüftimplantate unterschiedlich dimensioniert sind. Die Anzahl der zylindrischen Öffnungen einer Schablone entspricht der Anzahl verschiedener Augmente, die für das dem Probeimplantat entsprechende Hüftimplantat vorgesehen sind. Beispielsweise können in Ausführungsformen, in denen für jedes Hüftimplantat vier verschiedene Augmente vorgesehen sind, die zu dem entsprechenden Probeimplantat gehörenden Schablonen vier zylindrische Öffnungen aufweisen, wie in den Fig. 1 und 3 dargestellt. Die Schablonen können, wie in Fig. 1 gezeigt, Beschriftungen aufweisen, die das Hüftimplantat, dem die Schablone zugeordnet ist, sowie die Augmente, denen die einzelnen zylindrischen Öffnungen der Schablone zugeordnet sind, bezeichnen.

Das Hüftimplantatsystem kann außerdem Knochenfräser unterschiedlicher Größe umfassen, wobei jedem der Augmente ein von seinen Abmessungen her passender Knochenfräser zugeordnet ist.

Das Hüftimplantatsystem kann außerdem Positionierungsmittel ähnlich zu dem in Fig. 6 dargestellten Positionierungsmittel 600 unterschiedlicher Größe umfassen, die sich hinsichtlich der Länge des Gewindes 604 unterscheiden. Dadurch kann, wie unten genauer beschrieben, ein Positionierungsmittel mit einer für die individuelle Anatomie des Patienten geeigneten Größe ausgewählt werden, so dass einerseits eine bikortikale Befestigung des Positionierungsmittels 600 am Knochen erreicht werden kann, aber andererseits die Spitze des Gewindes 604 nicht zu weit aus dem Knochen herausragt, was zur Verletzungen des Gewebes des Patienten führen könnte.

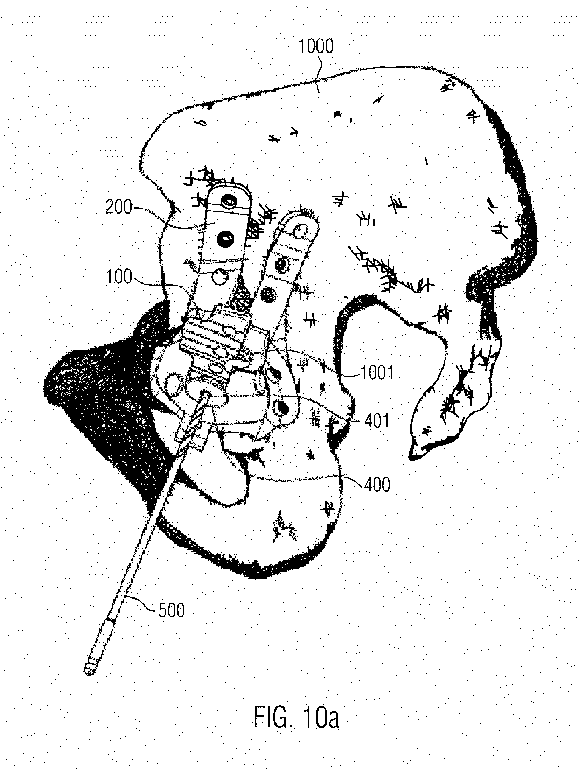

Im Folgenden wird die Anwendung der oben beschriebenen Komponenten einer erfindungsgemäßen Vorrichtung mit Bezug auf die Fig. 10A bis 10G beschrieben. Der Übersichtlichkeit halber wurden in den Fig. 10A bis 10G einige Bezugszeichen weggelassen.

Fig. 10A zeigt eine Ansicht eines Hüftknochens 1000 eines Patienten, an dem das oben mit Bezug auf Fig. 2 beschriebene Probehüftimplantat 200 angebracht ist. Um ein Verrutschen des Probehüftimplantats 200 zu vermeiden, kann das Probehüftimplantat 200 mit Hilfe einer durch eine der Bohrungen 210 des Probehüftimplantats 200 geführten Knochenschraube am Knochen 1000 fixiert werden. Vor dem Anbringen des Probehüftimplantats 200 am Knochen 1000 können bekannte Maßnahmen durchgeführt werden, um den Knochen 1000 vorzubereiten, beispielsweise kann das Azetabulum mit einem Knochenfräser ähnlich dem in Fig. 8 dargestellten Knochenfräser 800 angefräst werden.

Nach dem Anbringen des Probehüftimplantats 200 am Knochen 1000 kann die Schablone 100 mit Hilfe einer durch die Öffnung 115 der Schablone 100 geführten und in die Gewindeöffnung 206 des Probehüftimplantats 200 eingeschraubten Schraube 1001 am Probehüftimplantat 200 befestigt werden.

Vor oder nach dem Befestigen der Schablone 100 am Probehüftimplantat 200 kann eine für den Patienten passende Größe eines Augments bestimmt werden. In manchen Ausführungsformen können zu diesem Zweck an dem Probehüftimplantat 200 Markierungen angebracht sein, die den Konturen der verschieden großen Augmente entsprechen. Die Auswahl einer geeigneten Augmentgröße kann dann durch Vergleich der Markierungen am Probehüftimplantat mit den am Knochen 1000 vorhandenen Defekten erfolgen.

Anschließend kann die Bohrerführung 400 in diejenige der zylindrischen Öffnungen 101, 102, 103, 104 der Schablone 100, die der ausgewählten Augmentgröße zugeordnet ist, eingesetzt werden. In Fig. 10A ist die Bohrerführung 400 in die zylindrische Öffnung 104 der Schablone 100 eingesetzt dargestellt. Anschließend kann der Knochen 1000 mit Hilfe des Knochenbohrers 500 vorgebohrt werden. Zu diesem Zweck kann der Knochenbohrer 500 in den Kanal 401 der Bohrerführung 400 eingesetzt werden und mit Hilfe eines in Fig. 10A nicht dargestellten Antriebs in Rotation versetzt werden. Der Bohrvorgang kann solange durchgeführt werden, bis der Bohrer 500 auf der dem Probeimplantat 200 gegenüberliegenden Seite aus dem Knochen 1000 austritt. Anschließend können die Bohrerführung 400 und der Knochenbohrer 500 entfernt werden.

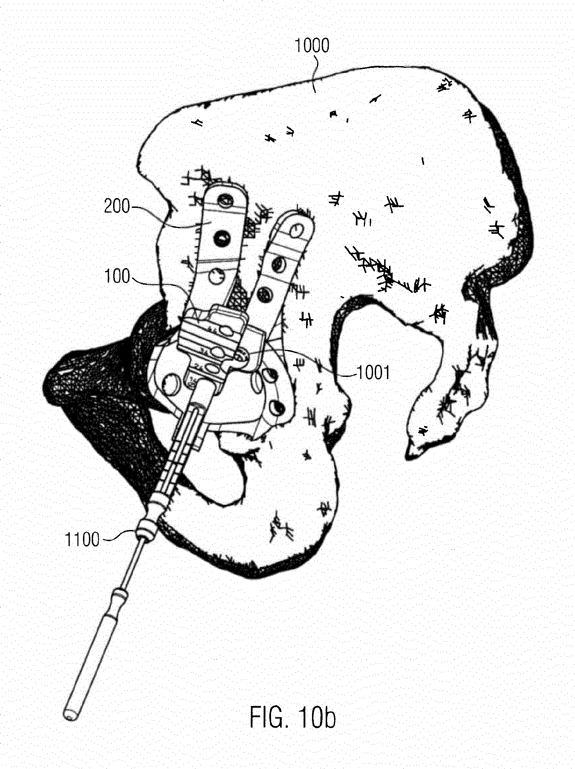

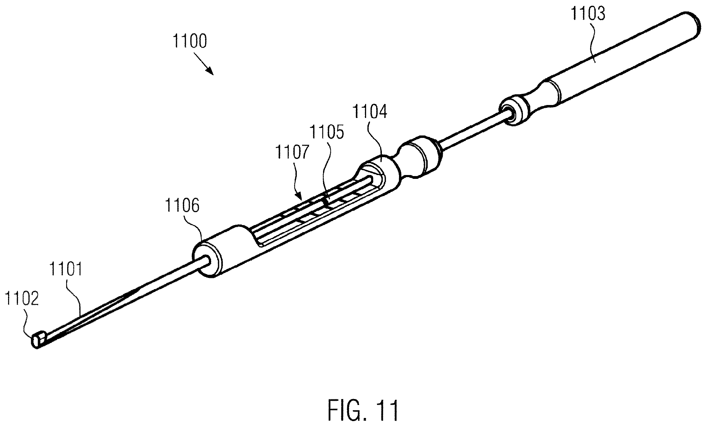

Danach kann, wie in Fig. 10B dargestellt, eine für den Patienten geeignete Größe des Positionierungsmittels 600 bestimmt werden. Zu diesem Zweck kann eine Tiefenmesslehre 1100 verwendet werden, die in Fig. 11 genauer dargestellt ist.

Die Tiefenmesslehre 1100 umfasst einen Schieber 1101 mit einer Markierung 1105. An einem Ende des Schiebers 1101 befindet sich ein Haken 1102. Am anderen Ende des Schiebers 1101 befindet sich ein Handgriff 1103. Die Tiefenmesslehre 1100 umfasst außerdem eine Stange 1104 mit einem Anschlag 1106 zur Auflage auf den Stufen 109, 110, 111, 112 der Schablone 100. An der Stange 1104 ist eine Skala 1107 angebracht. Der Schieber 1101 kann durch Betätigen des Handgriffs 1103 entlang der Stange 1104 verschoben werden und mit Hilfe der Markierung 1105 und der Skala 1107 kann ein Messwert für den Abstand zwischen dem Haken 1102 und dem Anschlag 1106 abgelesen werden.

Zum Bestimmen der benötigten Größe des Positionierungsmittels 600 kann das vom Handgriff 1103 abgewandte Ende des Schiebers 1101, an dem sich der Haken 1102 befindet, in die der gewählten Augmentgröße zugeordnete zylindrische Öffnung der Schablone 100 eingeführt werden. In dem in Fig. 10B dargestellten Beispiel ist dies die zylindrische Öffnung 104. Anschließend wird der Anschlag 1106 der Stange 1104 an der der zylindrischen Öffnung entsprechenden Stufe 112 angesetzt und durch Verschieben des Schiebers 1101 mit Hilfe des Handgriffs 1103 die dem Probehüftimplantat 200 gegenüberliegende Seite des Knochens 1000 mit dem Haken 1102 ertastet. Dann kann durch Ablesen der Position der Markierung 1105 relativ zur Skala 1107 der Abstand der gegenüberliegenden Seite des Knochens 1000 von der Stufe 112 bestimmt werden. Dann kann ein Positionierungsmittel 600 mit einer an den gemessenen Wert angepassten, zum Erreichen einer bikortikalen Fixierung geeigneten Länge ausgewählt werden.

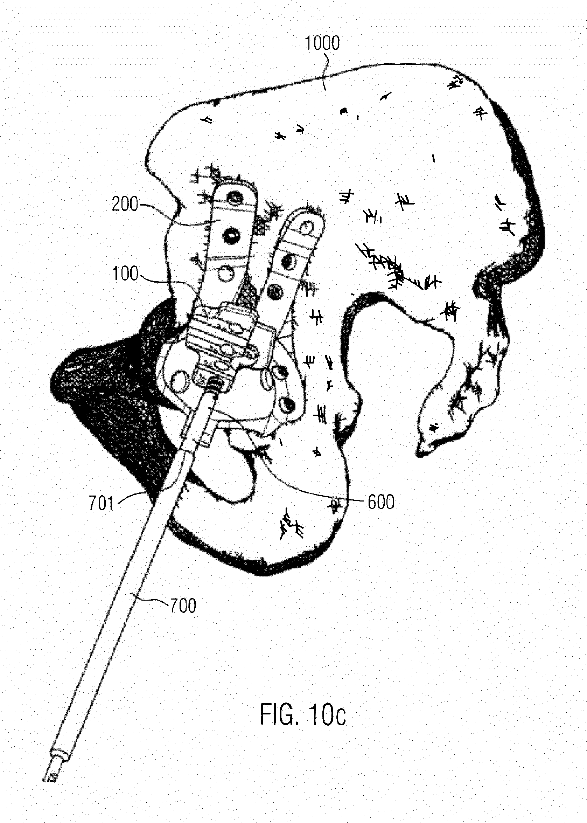

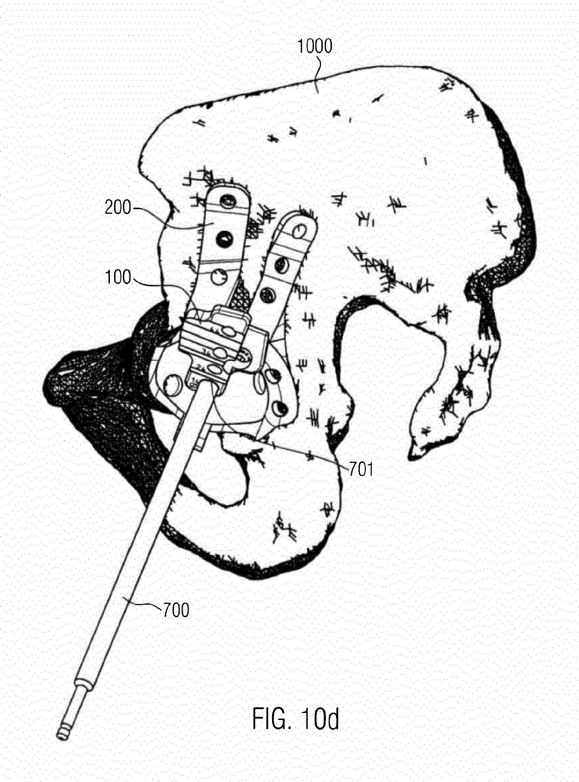

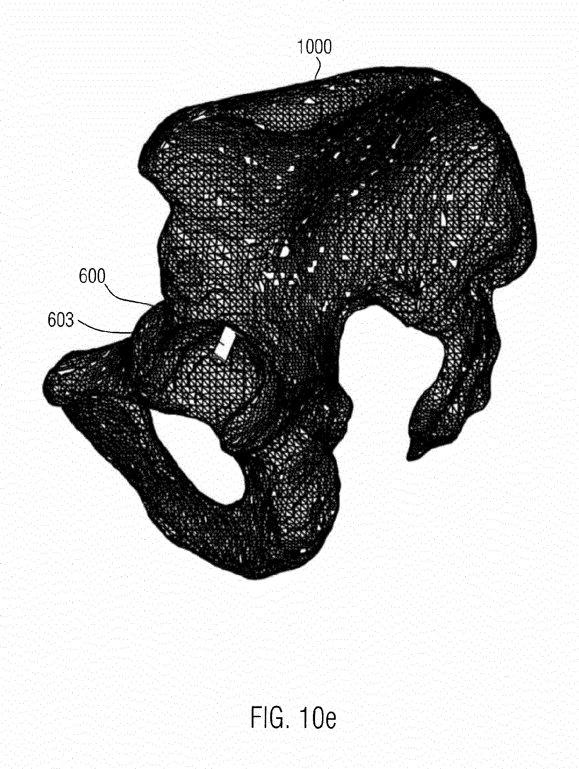

Anschließend kann, wie in Fig. 10C dargestellt, das Positionierungsmittel 600 in die der gewählten Augmentgröße zugeordnete zylindrische Öffnung 104 eingesetzt werden und mit Hilfe des Werkzeugs 700 in den Knochen 1000 eingeschraubt werden. Zu diesem Zweck kann das Werkzeug 700 mit Hilfe eines in Fig. 10C nicht dargestellten Antriebs in Rotation versetzt werden. Das Positionierungsmittel 600 kann solange eingeschraubt werden, bis der Anschlag 701 des Werkzeugs 700 an der Stufe 112 der Schablone 100, durch die die der gewählten Augmentgröße zugordnete zylindrische Öffnung 104 verläuft, anstößt, wie in Fig. 10D dargestellt. Das distale Ende 603 des Positionierungsmittels 600 befindet sich dann relativ zu dem Probehüftimplantat 200 an einer Position, die durch die Geometrie des Probehüftimplantats 200, der Schablone 100 und des Werkzeugs 700 festgelegt ist. Da das Positionierungsmittel 600 mit dem Knochen 1000 verschraubt ist, bleibt das distale Ende 603 des Positionierungsmittels 600 an dieser Position, wenn, wie in Fig. 10E dargestellt, nach dem Anbringen des Positionierungsmittels 600 am Knochen das Probehüftimplantat 200 und die Schablone 100 entfernt werden.

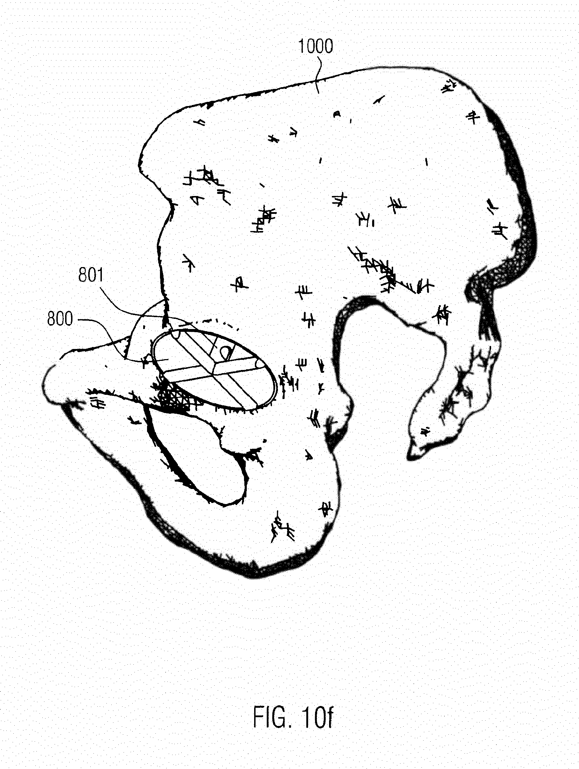

Anschließend kann, wie in Fig. 10F dargestellt, der Knochen 1000 mit Hilfe des Knochenfräsers 800 bearbeitet werden, wobei, im Fall mehrerer verschieden großer Knochenfräser, der der gewählten Augmentgröße zugeordnete Knochenfräser verwendet wird. Der Führungsabschnitt 601 des Positionierungsmittels 600 wird durch die Öffnung 806 am Pol des Bearbeitungsabschnitts 803 in den Aufnahmeabschnitt 801 des Knochenfräsers 800 eingesetzt. Dann kann der Knochenfräser 800 mit Hilfe eines in Fig. 10F nicht dargestellten Antriebs in Rotation versetzt werden, wodurch solange Material vom Knochen 1000 abgetragen wird, bis das distale Ende 603 des Positionierungsmittels am Anschlag 802 des Knochenfräsers 800 anstößt, wodurch ein weiteres Entfernen von Knochenmaterial durch den Knochenfräser 800 verhindert wird.

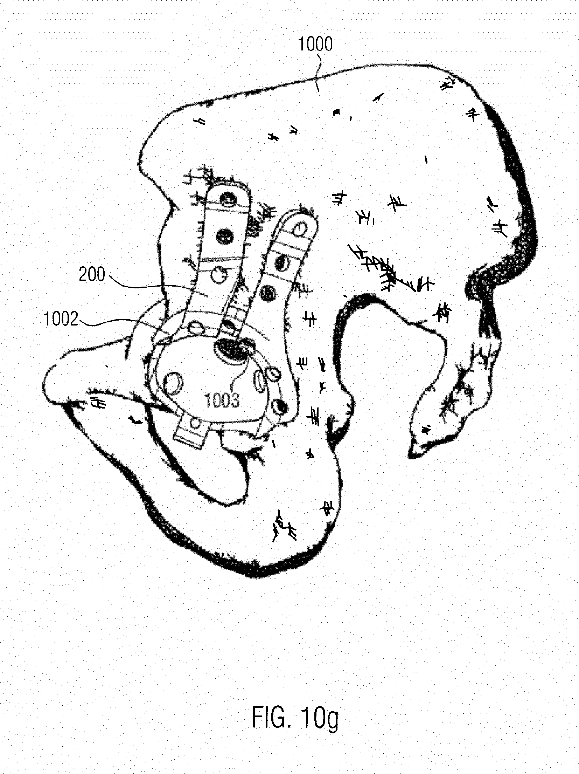

Anschließend kann das Positionierungsmittel 600 aus dem Knochen 1000 herausgeschraubt werden, und es kann, wie in Fig. 10G dargestellt, mit Hilfe des Probehüftimplantats 200 und eines an dem Probehüftimplantat 200 angebrachten Probeaugments 1002, das im Wesentlichen die gleiche Form wie das endgültige Augment der gewählten Größe hat, überprüft werden, ob eine korrekte Positionierung des endgültigen Hüftimplantats mit dem endgültigen Augment am Knochen 1000 erreicht werden kann. Ist dies der Fall, können das endgültige Hüftimplantat und das endgültige Augment am Knochen 1000 des Patienten befestigt werden.

## Patentansprüche

1. Vorrichtung zum Anbringen eines Positionierungsmittels (600) an einem Knochen (1000) eines Patienten in einer vorgegebenen Position relativ zu einem Hüftimplantat (900), umfassend:
ein Probehüftimplantat (200) mit einer Auflagefläche (201) zur Auflage auf dem Knochen (1000) des Patienten; und
eine Schablone (100), die an dem Probehüftimplantat (200) befestigbar ist und mindestens eine zylindrische Öffnung (101, 102, 103, 104) mit einer Längsachse (105, 106, 107, 108) aufweist;
wobei das Probehüftimplantat (200) eine solche Form hat, dass jede Gerade, die durch eine der mindestens einen zylindrischen Öffnung (101, 102, 103, 104) verläuft und zur Längsachse (105, 106, 107, 108) der jeweiligen Öffnung (101, 102, 103, 104) parallel ist, das Probehüftimplantat (200) nicht schneidet, wenn die Schablone (100) an dem Probehüftimplantat (200) befestigt ist;
**dadurch gekennzeichnet, dass** das Probehüftimplantat (200) einen Abschnitt (202) mit einer allgemeinen Form einer Kugelkalottenschale und einen Randabschnitt (203) umfasst und die Schablone (100) auf einer Seite, die dem Probehüftimplantat (200) zugewandt ist, wenn die Schablone (100) an dem Probehüftimplantat (200) befestigt ist, eine Oberfläche mit einem Abschnitt (113), der eine zu dem Randabschnitt (203) komplementäre Form hat, aufweist; und
das Probehüftimplantat (200) einen schlitzförmigen Einschnitt (204) aufweist, der sich durch den Abschnitt (202) mit der allgemeinen Form einer Kugelkalottenschale und den Randabschnitt (203) erstreckt, wobei für mindestens eine der mindestens einen zylindrischen Öffnung (101, 102, 103, 104) gilt, dass jede Gerade, die durch die Öffnung verläuft und zur Längsachse der Öffnung parallel ist, durch den schlitzförmigen Einschnitt (104) verläuft, wenn die Schablone (100) an dem Probehüftimplantat (200) befestigt ist.

2. Vorrichtung gemäß Anspruch 1, wobei die Schablone (100) mehrere zylindrische Öffnungen (101, 102, 103, 104) aufweist, die in einer Reihe angeordnet sind.

3. Vorrichtung gemäß Anspruch 2, wobei die Längsachsen (105, 106, 107, 108) der zylindrischen Öffnungen (101, 102, 103, 104) zueinander parallel sind.

4. Vorrichtung gemäß Anspruch 2 oder 3, wobei die Schablone (100) auf einer Seite, die von dem Probehüftimplantat (200) abgewandt ist, wenn die Schablone (100) an dem Probehüftimplantat (200) befestigt ist, eine abgestufte Oberfläche mit mehreren Stufen (109, 110, 111, 112) hat, wobei sich durch jede der Stufen (109, 110, 111, 112) eine der mehreren zylinderförmigen Öffnungen (101, 102, 103, 104) erstreckt.

5. Vorrichtung gemäß Anspruch 4, wobei zumindest ein Teil von jeder der Stufen (109, 110, 111, 112) eine Oberfläche hat, die zu der Längsachse (105, 106, 107, 108) der zylindrischen Öffnung (101, 102, 103, 104), die sich durch die jeweilige Stufe erstreckt, senkrecht ist.

6. Vorrichtung gemäß einem der Ansprüche 1 bis 5, wobei:
das Probehüftimplantat (200) auf einer ersten Seite des schlitzförmigen Einschnitts (204) eine Öffnung (205) im Randabschnitt (203) aufweist und die Schablone (100) einen zu der Öffnung (205) im Randabschnitt (203) komplementären Vorsprung (114) aufweist, der in die Öffnung (205) im Randabschnitt (203) eingreift, wenn die Schablone (100) an dem Probehüftimplantat (200) befestigt ist;
das Probehüftimplantat (200) auf einer zweiten Seite des schlitzförmigen Einschnitts (204), die der ersten Seite gegenüberliegt eine Gewindeöffnung (206) im Randabschnitt (203) mit einem Innengewinde aufweist und die Schablone (100) eine von der genannten mindestens einen zylindrischen Öffnung (101, 102, 103, 104) separate Öffnung (115) aufweist, die mit der Gewindeöffnung (206) im Randabschnitt (203) fluchtet, wenn die Schablone (100) an dem Probehüftimplantat (200) befestigt ist; und
die Vorrichtung zusätzlich eine Schraube (1001) mit einem zu dem Innengewinde in der Gewindeöffnung (206) im Randabschnitt (203) des Probehüftimplantats (200) komplementären Außengewinde zum Verschrauben der Schablone (100) mit dem Probehüftimplantat (200) umfasst.

7. Vorrichtung gemäß einem der Ansprüche 1 bis 6, die zusätzlich eine Bohrerführung (400) umfasst, die durch die mindestens eine zylindrische Öffnung (101, 102, 103, 104) der Schablone (100) steckbar ist und einen Kanal (401) aufweist, durch den ein Knochenbohrer (500) führbar ist.

8. Vorrichtung gemäß einem der Ansprüche 1 bis 7, die zusätzlich das Positionierungsmittel (600) umfasst, wobei das Positionierungsmittel (600) einen zylindrischen Führungsabschnitt (601) mit einer Längsachse (602) aufweist, der durch die mindestens eine zylindrische Öffnung (101, 102, 103, 104) der Schablone (100) führbar ist, wobei der Führungsabschnitt (601) relativ zur Schablone (100) entlang der Längsachse (105, 106, 107, 108) der zylindrischen Öffnung (101, 102, 103, 104) beweglich ist und die Längsachse (602) des Führungsabschnitts (601) entlang der Längsachse (105, 106, 107, 108) der zylindrischen Öffnung (101, 102, 103, 104) ausgerichtet ist.

9. Vorrichtung gemäß Anspruch 8, die zusätzlich ein Werkzeug (700) zum Befestigen des Positionierungsmittels (600) am Knochen (1000) des Patienten durch eine der mindestens einen zylindrischen Öffnung (101, 102, 103, 104) der Schablone (100) hindurch umfasst, wobei das Werkzeug (700) einen Anschlag (701) zum Festlegen eines Abstands zwischen einem distalen Ende (603) des am Knochen (1000) des Patienten befestigten Positionierungsmittels (600) und dem vom Probeimplantat (200) abgewandten Ende der zylindrischen Öffnung (101, 102, 103, 104) der Schablone (100), durch die hindurch das Positionierungsmittel (600) am Knochen (1000) des Patienten befestigt wird, umfasst.

10. Vorrichtung gemäß Anspruch 9, wobei das Positionierungsmittel (600) eine Knochenschraube umfasst, das Werkzeug (700) zum Befestigen des Positionierungsmittels (600) am Knochen (1000) des Patienten einen Schraubendreher umfasst und der Anschlag (701) des Werkzeugs (700) zum Befestigen des Positionierungsmittels (600) durch einen Abschnitt (702) eines Schafts (704) des Schraubendrehers (700), der einen größeren Durchmesser aufweist als die mindestens eine zylindrische Öffnung (101, 102, 103, 104) der Schablone (100), bereitgestellt wird.

11. Vorrichtung zum Bearbeiten eines Knochens (1000) eines Patienten bei einer Implantation eines Hüftimplantats (900), umfassend:
eine Vorrichtung zum Anbringen eines Positionierungsmittels (600) an einem Knochen (1000) eines Patienten gemäß einem der Ansprüche 8 bis 10; und
mindestens einen Knochenfräser (800), der einen Aufnahmeabschnitt (801) für den Führungsabschnitt (601) des Positionierungsmittels (600) aufweist, wobei der Führungsabschnitt (601) in den Aufnahmeabschnitt (801) einsetzbar ist, und der Aufnahmeabschnitt (801) so ausgebildet ist, dass eine Drehachse (811) des Knochenfräsers (800) entlang der Längsachse (602) des Führungsabschnitts (601) ausgerichtet ist und der Knochenfräser (800) um die Längsache (602) des Führungsabschnitts (601) drehbar und entlang der Längsachse (602) des Führungsabschnitts (601) verschiebbar ist, wenn der Führungsabschnitt (601) in den Aufnahmeabschnitt (801) des Knochenfräsers (800) eingesetzt ist.

12. Vorrichtung gemäß Anspruch 11, wobei der Aufnahmeabschnitt (801) des Knochenfräsers (800) einen Anschlag (802) für das distale Ende (603) des Positionierungsmittels (600) umfasst.

13. Hüftimplantatsystem mit:
einer Vorrichtung zum Bearbeiten eines Knochens (1000) eines Patienten gemäß Anspruch 11 oder 12;
einem Hüftimplantat (900) zur Aufnahme einer künstlichen Gelenkpfanne, das eine Auflagefläche (901) zur Auflage auf dem Knochen (1000) des Patienten umfasst, die eine Form hat, die der Form der Auflagefläche (201) des Probehüftimplantats (200) entspricht; und
mindestens ein Augment (902), das an dem Hüftimplantat (900) befestigbar ist, wobei das Augment (902) auf einer Seite, die von dem Hüftimplantat (900) abgewandt ist, wenn das Augment (902) an dem Hüftimplantat (900) befestigt ist, eine Oberfläche (903) mit einer Form, die zu einer Form eines Bearbeitungsabschnitts (803) von einem des mindestens einen Knochenfräsers (800) korrespondiert, aufweist.

14. Hüftimplantatsystem gemäß Anspruch 13, wobei die Schablone (100) mehrere zylindrische Öffnungen (101, 102, 103, 104) aufweist, das Hüftimplantatsystem mehrere verschieden große Augmente (902) umfasst, und jeder von dem mindestens einen Knochenfräser (800) einen Bearbeitungsabschnitt (803) mit einer allgemein sphärischen Form aufweist;
wobei jedem Augment (902) eine der zylindrischen Öffnungen (101, 102, 103, 104) der Schablone (100) und einer von dem mindestens einen Knochenfräser (800) zugeordnet ist; und
wobei jedes Augment (902) auf der Seite, die von dem Hüftimplantat (900) abgewandt ist, wenn das Augment (902) an dem Hüftimplantat (900) befestigt ist, eine allgemein sphärische Oberfläche (903) aufweist, wobei ein Radius der allgemein sphärischen Oberfläche (903) einem Radius des allgemein sphärischen Bearbeitungsabschnitts (803) des einen von dem mindestens einen Knochenfräser (800), der dem Augment (902) zugeordnet ist, entspricht.

## Claims

1. Device for attaching a positioning device (600) to a bone (1000) of a patient at a predetermined position relative to a hip implant (900) comprising:
a trial hip implant (200) with a support surface (201) for resting on said bone (1000) of the patient; and
a template (100) that can be secured to said trial hip implant (200) and has at least one cylindrical opening (101, 102, 103, 104) with a longitudinal axis (105, 106, 107, 108);
wherein said trial hip implant (200) is shaped such that any straight line which runs through one of said at least one cylindrical opening (101, 102, 103, 104) and is parallel to said longitudinal axis (105, 106, 107, 108) of said respective opening (101, 102, 103, 104) does not intersect said trial hip implant (200) when said template (100) is secured to said trial hip implant (200);
**characterized in that** said trial hip implant (200) comprises a section (202) having a general shape of a spherical dome shell and an edge section (203), and said template (100) on one side facing said trial hip implant (200) when said template (100) is secured to said trial hip implant (200) has a surface with a section (113) having a shape that is complementary to said edge section (203); and
said trial hip implant (200) has a slit-shaped notch (204) that extends through said section (202) having the general shape of a spherical dome shell and said edge section (203), wherein it is for at least one of said at least one cylindrical opening (101, 102, 103, 104) true that any straight line extending through said opening and being parallel to the longitudinal axis of said opening extends through said slit-shaped notch (104) when said template (100) is secured to said trial hip implant (200).

2. Device according to claim 1, wherein said template (100) comprises several cylindrical openings (101, 102, 103, 104) that are arranged in a row.

3. Device according to claim 2, wherein said longitudinal axes (105, 106, 107, 108) of said cylindrical openings (101, 102, 103, 104) are parallel to each other.

4. Device according to claim 2 or 3, wherein said template (100) on a side facing away from said trial hip implant (200) when said template (100) is secured to said trial hip implant (200) has a stepped surface with several steps (109, 110, 111, 112), wherein one of said several cylindrical openings (101, 102, 103, 104) extends through each of said steps (109, 110, 111, 112).

5. Device according to claim 4, wherein at least a portion of each of said steps (109, 110, 111, 112) has a surface which is perpendicular to said longitudinal axis (105, 106, 107, 108) of said cylindrical opening (101, 102, 103, 104) extending through said respective step.

6. Device according to any of claims 1 to 5, wherein:
said trial hip implant (200) on a first side of said slit-shaped notch (204) comprises an opening (205) in said edge section (203) and said template (100) comprises a protrusion (114) that is complementary to said opening (205) in said edge section (203) and engages in said opening (205) in said edge section (203) when said template (100) is secured to said trial hip implant (200);
said trial hip implant (200) on a second side of said slit-shaped notch (204) being disposed opposite to said first side comprises a threaded opening (206) in said edge section (203) having an internal thread, and said template (100) comprises an opening (115) that is separate from said at least one cylindrical opening (101, 102, 103, 104) and is flush with said threaded opening (206) in said edge section (203) when said template (100) is secured to said trial hip implant (200); and
said device additionally comprises a screw (1001) with an external thread that is complementary to said internal thread in said threaded opening (206) in said edge section (203) of said trial hip implant (200) for screwing said template (100) to said trial hip implant (200).

7. Device according to any of claims 1 to 6, additionally comprising a drill guide (400) which is insertable through said at least one cylindrical opening (101, 102, 103, 104) of said template (100) and comprises a channel (401) through which a bone drill (500) can be guided.

8. Device according to any of claims 1 to 7, additionally comprising said positioning device (600) wherein said positioning device (600) comprises a cylindrical guide section (601) having a longitudinal axis (602) which can be guided through said at least one cylindrical opening (101, 102, 103, 104) of said template (100); wherein said guide section (601) is moveable relative to said template (100) along said longitudinal axis (105, 106, 107, 108) of said cylindrical opening (101, 102, 103, 104) and said longitudinal axis (602) of said guide section (601) is aligned along said longitudinal axis (105, 106, 107, 108) of said cylindrical opening (101, 102, 103, 104).

9. Device according to claim 8, additionally comprising a tool (700) for securing said positioning device (600) to said bone (1000) of the patient through one of said at least one cylindrical opening (101, 102, 103, 104) of said template (100); wherein said tool (700) comprises a stop (701) for defining a distance between a distal end (603) of said positioning device (600) secured to said bone (1000) of the patient and the end of said cylindrical opening (101, 102, 103, 104) of said template (100) that faces away from said trial implant (200) through which said positioning device (600) is secured to said bone (1000) of the patient.

10. Device according to claim 9, wherein said positioning device (600) comprises a bone screw, said tool (700) for securing said positioning device (600) to said bone (1000) of the patient comprises a screwdriver, and said stop (701) of said tool (700) for securing said positioning device (600) is provided by a section (702) of a shank (704) of said screwdriver (700) which is larger in diameter than said at least one cylindrical opening (101, 102, 103, 104) of said template (100).

11. Device for treating a bone (1000) of a patient when implanting a hip implant (900), comprising:
a device for attaching a positioning device (600) to a bone (1000) of a patient according to any of claims 8 to 10; and
at least one bone milling device (800) comprising a receiving section (801) for said guide section (601) of said positioning device (600), wherein said guide section (601) can be inserted into said receiving section (801), and said receiving section (801) is formed such that an axis of rotation (811) of said bone milling device (800) is aligned along said longitudinal axis (602) of said guide section (601), and said bone milling device (800) is rotatable about said longitudinal axis (602) of said guide section (601) and is moveable along said longitudinal axis (602) of said guide section (601) when said guide section (601) is inserted into said receiving section (801) of said bone milling device (800).

12. Device according to claim 11, where said receiving section (801) of said bone milling device (800) comprises a stop (802) for said distal end (603) of said positioning device (600).

13. Hip implant system with:
a device for treating a bone (1000) of a patient according to claim 11 or 12;
a hip implant (900) for receiving an artificial joint socket and comprising a support surface (901) for resting on said bone (1000) of the patient which has a shape corresponding to the shape of said support surface (201) of said trial hip implant (200); and
at least one augment (902) that can be secured to said hip implant (900), wherein said augment (902) on a side facing away from said hip implant (900) when said augment (902) is secured to said hip implant (900) comprises a surface (903) having a shape corresponding to a shape of a treatment section (803) of one of said at least one a bone milling device (800).

14. Hip implant system according to claim 13, wherein said template (100) comprises a plurality of cylindrical openings (101, 102, 103, 104), said hip implant system comprises a plurality of augments (902) of different sizes, and each of said at least one bone milling device (800) comprises a treatment section (803) having a generally spherical shape;
wherein each augment (902) is associated with one of said cylindrical openings (101, 102, 103, 104) of said template (100) and one of said at least one bone milling device (800); and
wherein each augment (902) on the side facing away from said hip implant (900) when said augment (902) is secured to said hip implant (900) has a generally spherical surface (903), wherein a radius of said generally spherical surface (903) corresponds to a radius of said generally spherical treatment section (803) of said one of said at least one bone milling device (800) which is associated with said augment (902).

## Revendications

1. Dispositif pour la mise en place d'un moyen de positionnement (600) sur un os (1000) d'un patient, dans une position prédéterminée par rapport à un implant de hanche (900), comprenant :
un implant de hanche d'essai (200) avec une surface d'appui (201) destinée à s'appuyer sur l'os (1000) du patient ; et
un gabarit (100), qui peut être fixé à l'implant de hanche d'essai (200), et présente au moins une ouverture cylindrique (101, 102, 103, 104) avec un axe longitudinal (105, 106, 107, 108) ;
dispositif
dans lequel l'implant de hanche d'essai (200) présente une forme telle, que chaque droite, qui s'étend à travers l'une desdites ouvertures cylindriques (101, 102, 103, 104) au nombre d'au moins une, et qui est parallèle à l'axe longitudinal (105, 106, 107, 108) de l'ouverture (101, 102, 103, 104) respective, ne coupe pas l'implant de hanche d'essai (200) lorsque le gabarit (100) est fixé à l'implant de hanche d'essai (200) ;
**caractérisé en ce que** l'implant de hanche d'essai (200) comprend un tronçon (202) avec une forme générale de cavité en calotte sphérique et un tronçon de bordure (203), et le gabarit (100) présente sur un côté, qui est dirigé vers l'implant de hanche d'essai (200) lorsque le gabarit (100) est fixé à l'implant de hanche d'essai (200), une surface avec un tronçon (113) possédant une forme complémentaire au tronçon de bordure (203) ; et
l'implant de hanche d'essai (200) présente une incision (204) en forme de fente, qui s'étend à travers le tronçon (202) avec la forme générale de cavité en calotte sphérique, et à travers le tronçon de bordure (203), la configuration étant telle, que pour au moins l'une desdites ouvertures cylindriques (101, 102, 103, 104) au nombre d'au moins une, chaque droite, qui s'étend à travers l'ouverture et est parallèle à l'axe longitudinal de l'ouverture, s'étend à travers l'incision (204) en forme de fente lorsque le gabarit (100) est fixé à l'implant de hanche d'essai (200).

2. Dispositif selon la revendication 1, dans lequel le gabarit (100) présente plusieurs ouvertures cylindriques (101, 102, 103, 104), qui sont agencées sur une rangée.

3. Dispositif selon la revendication 2, dans lequel les axes longitudinaux (105, 106, 107, 108) des ouvertures cylindriques (101, 102, 103, 104) sont mutuellement parallèles.

4. Dispositif selon la revendication 2 ou la revendication 3, dans lequel le gabarit (100) possède sur un côté, qui est opposé à celui dirigé vers l'implant de hanche d'essai (200) lorsque le gabarit (100) est fixé à l'implant de hanche d'essai (200), une surface étagée avec plusieurs étagements (109, 110, 111, 112), la configuration étant telle qu'à travers chacun des étagements (109, 110, 111, 112) s'étende l'une desdites plusieurs ouvertures cylindriques (101, 102, 103, 104).

5. Dispositif selon la revendication 4, dans lequel au moins une partie de chacun des étagements (109, 110, 111, 112) possède une surface qui est perpendiculaire à l'axe longitudinal (105, 106, 107, 108) de l'ouverture cylindrique (101, 102, 103, 104), qui s'étend à travers l'étagement respectif considéré.

6. Dispositif selon l'une des revendications 1 à 5, dans lequel :
l'implant de hanche d'essai (200) présente, sur un premier côté de l'incision (204) en forme de fente, une ouverture (205) dans le tronçon de bordure (203), et le gabarit (100) présente une protubérance (114), qui est complémentaire à l'ouverture (205) dans le tronçon de bordure (203), et qui s'engage dans l'ouverture (205) dans le tronçon de bordure (203) lorsque le gabarit (100) est fixé à l'implant de hanche d'essai (200) ;
l'implant de hanche d'essai (200) présente, sur un deuxième côté de l'incision (204) en forme de fente, qui est opposée au dit premier côté, une ouverture taraudée (206) dans le tronçon de bordure (203), avec un filetage intérieur, et le gabarit (100) présente une ouverture (115), qui est séparée de ladite au moins une des ouvertures cylindriques (101, 102, 103, 104) et qui est alignée avec l'ouverture taraudée (206) dans le tronçon de bordure (203) lorsque le gabarit (100) est fixé à l'implant de hanche d'essai (200) ; et
le dispositif comprend, en outre, une vis (1001) avec un filetage extérieur complémentaire au filetage intérieur de l'ouverture taraudée (206) dans le tronçon de bordure (203) de l'implant de hanche d'essai (200), pour visser le gabarit (100) sur l'implant de hanche d'essai (200).

7. Dispositif selon l'une des revendications 1 à 6, comprenant, en outre, un canon de perçage (400), qui peut être engagé dans ladite au moins une des ouvertures cylindriques (101, 102, 103, 104) du gabarit (100), et présente un canal (401) permettant d'assurer le guidage d'un foret à os (500).

8. Dispositif selon l'une des revendications 1 à 7, comprenant, en outre, le moyen de positionnement (600), et dans lequel le moyen de positionnement (600) présente un tronçon de guidage cylindrique (601) avec un axe longitudinal (602), qui peut être guidé à travers ladite au moins une ouverture cylindrique (101, 102, 103, 104) du gabarit (100), et dans lequel le tronçon de guidage (601) est mobile par rapport au gabarit (100) le long de l'axe longitudinal (105, 106, 107, 108) de l'ouverture cylindrique (101, 102, 103, 104), et l'axe longitudinal (602) du tronçon de guidage (601) est orienté le long de l'axe longitudinal (105, 106, 107, 108) de l'ouverture cylindrique (101, 102, 103, 104).

9. Dispositif selon la revendication 8, comprenant, en outre, un outil (700) pour fixer le moyen de positionnement (600) à l'os (1000) du patient à travers une des ouvertures cylindriques (101, 102, 103, 104) au nombre d'au moins une, du gabarit (100), dispositif dans lequel l'outil (700) comporte une butée (701) pour fixer une distance entre une extrémité distale (603) du moyen de positionnement (600) fixé à l'os (1000) du patient, et l'extrémité éloignée de l'implant de hanche d'essai (200), de l'ouverture cylindrique (101, 102, 103, 104) du gabarit (100), à travers laquelle le moyen de positionnement (600) est fixé à l'os (1000) du patient.

10. Dispositif selon la revendication 9, dans lequel le moyen de positionnement (600) comprend une vis à os, l'outil (700) pour fixer le moyen de positionnement (600) à l'os (1000) du patient comprend un tournevis, et la butée (701) de l'outil (700) pour fixer le moyen de positionnement (600) est formée par un tronçon (702) d'un corps (704) du tournevis (700), qui présente un diamètre plus grand que ladite au moins une ouverture cylindrique (101, 102, 103, 104) du gabarit (100) .

11. Dispositif pour usiner un os (1000) d'un patient lors de l'implantation d'un implant de hanche (900), comprenant :
un dispositif pour la mise en place d'un moyen de positionnement (600) sur un os (1000) d'un patient, selon l'une des revendications 8 à 10 ; et
au moins une fraise à os (800), qui présente un tronçon d'accueil (801) pour le tronçon de guidage (601) du moyen de positionnement (600), dispositif dans lequel le tronçon de guidage (601) peut être inséré dans le tronçon d'accueil (801), et le tronçon d'accueil (801) est réalisé de manière telle, qu'un axe de rotation (811) de la fraise à os (800) soit orienté le long de l'axe longitudinal (602) du tronçon de guidage (601), et que la fraise à os (800) soit rotative autour de l'axe longitudinal (602) du tronçon de guidage (601) et coulissante le long de l'axe longitudinal (602) du tronçon de guidage (601), lorsque le tronçon de guidage (601) est inséré dans le tronçon d'accueil (801) de la fraise à os (800).

12. Dispositif selon la revendication 11, dans lequel le tronçon d'accueil (801) de la fraise à os (800) comporte une butée (802) pour l'extrémité distale (603) du moyen de positionnement (600).

13. Système d'implant de hanche comprenant :
un dispositif pour l'usinage d'un os (1000) d'un patient, selon la revendication 11 ou la revendication 12 ;
un implant de hanche (900) destiné à accueillir une cupule d'articulation artificielle, qui comprend une surface d'appui (901) destinée s'appuyer sur l'os (1000) du patient, qui possède une forme correspondant à la forme de la surface d'appui (201) de l'implant de hanche d'essai (200) ; et
au moins une rehausse (902), qui peut être fixée à l'implant de hanche (900), la configuration étant telle que la rehausse (900) présente sur un côté, qui est éloigné de l'implant de hanche (900) lorsque la rehausse (902) est fixée à l'implant de hanche (900), une surface (903) avec une forme correspondant à une forme d'un tronçon d'usinage (803) de ladite au moins une fraise à os (800).

14. Système d'implant de hanche selon la revendication 13, dans lequel le gabarit (100) présente plusieurs ouvertures cylindriques (101, 102, 103, 104), le système d'implant de hanche comprend plusieurs rehausses (902) de grandeur différente, et chacune desdites fraises à os (800) au nombre d'au moins une, présente un tronçon d'usinage (803) d'une forme générale sphérique ;
système d'implant de hanche
dans lequel à chaque rehausse (902) est associée l'une des ouvertures cylindriques (101, 102, 103, 104) du gabarit (100), et l'une desdites fraises à os (800) au nombre d'au moins une ; et
dans lequel chaque rehausse (902) présente, sur le côté qui est éloigné de l'implant de hanche (900) lorsque la rehausse (902) est fixée à l'implant de hanche (900), une surface (903) de forme générale sphérique, un rayon de la surface (903) de forme générale sphérique correspondant à un rayon du tronçon d'usinage (803) de forme générale sphérique de l'une desdites fraises à os (800) au nombre d'au moins une, associée à la rehausse (902) .
